# EUROPÄISCHE PATENTSCHRIFT

(11) **EP 4 547 636 B1**
(45) Veröffentlichungstag und Bekanntmachung des Hinweises auf die Patenterteilung: **10.06.2026**
(21) Anmeldenummer: 22768695.3
(22) Anmeldetag: 22.08.2022
(51) Int. Cl.: C07C 41/54, C07C 43/315, C07C 45/51, C07C 47/11, C07C 47/12, C11B 9/00

(54) **VERFAHREN ZUR HERSTELLUNG VON RIECH- UND DUFTSTOFFEN AM DÜNNSCHICHTVERDAMPFER**
METHOD FOR PRODUCING ODORANTS AND FRAGRANCES ON A THIN-FILM EVAPORATOR
PROCÉDÉ DE PRODUCTION D'ODORISANTS ET DE PARFUMS SUR UN ÉVAPORATEUR À COUCHE MINCE

(30) Priorität: 30.06.2022 US 202263357164 P
(43) Veröffentlichungstag der Anmeldung: 07.05.2025
(73) Patentinhaber: Symrise AG, 37603 Holzminden (DE)
(72) Erfinder: HÖLSCHER, Bernd, 37620 Halle (DE); GERSTMANN, Frank, 60599 Frankfurt am Main (DE); BARGSTEN, Stefanie, 32839 Steinheim (DE); KOLOMEYER, Gennadiy, Jacksonville, 32246 (US); SINGER, Emilie, 37603 Holzminden (DE)
(74) Vertreter: Global IP Europe Patentanwaltskanzlei
(86) Internationale Anmeldenummer: PCT/EP2022/073310
(87) Internationale Veröffentlichungsnummer: WO 2024/002503

(56) Entgegenhaltungen:
- EP-B1- 0 992 477
- US-A- 5 561 209

## Beschreibung

### Gebiet der Erfindung

Die vorliegende Erfindung betrifft ein Verfahren zur Herstellung von Riechstoffen oder Duftstoffen unter Durchführung von thermischen Umlagerungsreaktionen chemischer Verbindungen unter milden und ressourcenschonenden Bedingungen in einem Dünnschichtverdampfer unter destillativen Bedingungen, sowie die Produkte, d. h. Verbindungen unmittelbar hervorgehend aus diesem Verfahren. Insbesondere betrifft die vorliegende Erfindung ein alternatives und neues Verfahren zur Herstellung von 3-(4-Isopropylcyclohexen-1-yl)propanal der Formel (I).

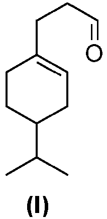

### Stand der Technik

Anforderungen an Herstellungsmethoden chemischer Produkte sind primär Wirtschaftlichkeit, hohe Ausbeute und Selektivität unter Einhaltung von allen sicherheitsrelevanten Aspekten. Eine material- und damit kosteneffiziente Herstellung liefert erheblichen Wettbewerbsvorteil gegenüber Mitbewerbern insbesondere im Hinblick auf den steigenden Bedarf und somit erhöhter Nachfrage nach bestimmten chemischen Produkten, wie beispielsweise Riech- oder Duftstoffen. In diesem Zusammenhang ist zu bedenken, dass ein effizienter Materialeinsatz anfallende Produktionskosten reduziert und gleichzeitig die Produktivität des Herstellungsprozesses steigern kann.

Eine Optimierung des Materialeinsatzes in der Produktion unter Berücksichtigung von Umweltgesichtspunkten stellt daher eine große Herausforderung bei der Herstellung chemischer Verbindungen sowie ihre Weiterverarbeitung, als Rohstoff, als chemisches Produkt, als Inhaltsstoff oder ähnlichem dar.

In diesem Kontext sollten chemische Herstellungsverfahren auch Aspekte der sogenannten grünen Chemie, also Umweltfreundlichkeit, berücksichtigen.

Ein weiterer entscheidender Gesichtspunkt ist daher Herstellungsverfahren durch den Einsatz von wiederaufbereiteten Reststoffen, nicht umgesetzten Stoffen, Abfallstoffen oder Nebenprodukten als Sekundärrohstoffe für weitere oder im Idealfall, als Ausgangstoff für das zugrundeliegende Verfahren als solches, möglichst materialeffizient zu gestalten, dabei die Umwelt zu schonen und gleichzeitig Produktions- und Entsorgungskosten zu senken. Ein solcher Wiedereinsatz von Ressourcen schont die vorhandenen Bestände und leistet somit einen entscheidenden Beitrag zur Nachhaltigkeit des Herstellungsprozesses während gleichzeitig anfallende Kosten reduziert werden können.

Darüber hinaus sind milde Reaktionsbedingungen aufgrund des geringen aufzuwendenden Energieaufwandes zu bevorzugen und führen bei chemischen Prozessen häufig zu steuerbareren und einheitlicheren Selektivitäten der Produkte.

Temperaturempfindliche Stoffe, wie beispielsweise viele Riechstoffe und/oder Aromastoffe dürfen nur kurzzeitig auf hohe Temperaturen erhitzt werden, um ungewollten thermischen Zersetzungsprozessen entgegenzuwirken. Klassische Destillationsverfahren führen in der Regel zu einer langen thermischen Belastung der zu destillierende Komponente. Dies kann negative Auswirkungen auf die Ausbeute als auch auf die Qualität des gewünschten chemischen Produktes haben, sofern dieses thermolabile Eigenschaften besitzt. Schnelle Destillationsverfahren mit zufriedenstellender Trennleistung und kurzen Verweilzeiten sind daher wünschenswert.

Schnelle Reaktionen und damit kurze Verweilzeiten können eine Steigerung der Raum-Zeit Ausbeute bewirken und reduzieren die thermische Belastung von chemischen Erzeugnissen, welche sich oft negativ auf die Qualität und Eigenschaften der Erzeugnisse auswirkt. Gründe hierfür können, wie bereits erwähnt, beispielsweise thermische Zersetzungen aufgrund von zu hoher thermischer Belastung sein.

Eine Möglichkeit innerhalb kurzer Verweilzeiten größere Mengen Ausgangsmaterial zu destillieren, ohne diese allzu großen thermischen Belastungen auszusetzen, besteht mittlerweile in der Verwendung von Dünnschichtverdampfern, in denen das zu verarbeitende Material als dünner Film auf die Verdampferinnenfläche aufgebracht wird, während nur sehr kurze Kontaktzeiten mit der beheizten Fläche bestehen.

Dünnschichtverdampfer eignen sich primär für die thermische Auftrennung eines Stoffgemisches durch Destillation, so finden Dünnschichtverdampfer unter anderem Verwendung in der Aufreinigung von Stoffgemischen.

So wird in beispielsweise in EP 3103538 A1 ein Dünnschichtverdampfer zum thermischen Trennen leichter flüchtiger Fraktionen von schwerer siedenden Rückständen in einem Stoffgemisch durch Eindampfen beschrieben, welcher mit einem geschlossenen, im Wesentlichen zylindrischen Reaktorbehälter, dessen Reaktorwandung mittels einer Heizeinrichtung beheizbar ist, mit einem Einlass für das aufzutrennende Einsatzmaterial, einem Auslass für die Rückstände und einem Abzug für die Brüden, sowie mit zumindest einer, mit einem Antriebsmittel verbundenen Antriebswellenanordnung, die zumindest eine Wischeinrichtung mit einem Rotor mit Wischerelementen für die Innenfläche der Reaktorwandung besitzt, ausgestaltet ist.

Seitens der Kosmetik-Industrie, Parfümerie und vergleichbaren Wirtschaftssektoren besteht beispielsweise ein steigender Bedarf an hochqualitativen Inhalts- und Wirkstoffen. Derartige Stoffe können beispielsweise Aroma- und Riech- oder Duftstoffe sein. Da Riechstoffe oft sehr spezifische Geruchsprofile aufweisen ist es daher besonders wünschenswert derartige Produkte möglichst rein und frei von Verunreinigungen, d. h. möglichst selektiv zu synthetisieren, da mögliche Verunreinigungen des Riechstoffes den charakteristischen Geruchseindruck verfälschen oder nachteilig beeinflussen können und unangenehme Nebengerüche verursachen können. Derartige Verunreinigungen können sich ferner aufgrund von chemischen Wechselwirkungen negativ auf die Stabilität der synthetisierten Riechstoffe auswirken und unangenehme Geruchsnoten hervorrufen oder den zugrundeliegenden Geruchseindruck verändern. Daher ist ein möglichst hoher Reinheitsgrad solcher Verbindungen gewünscht.

Zwar können Stoffgemische derzeit wie bereits oben erwähnt mittels Destillation, z. B. in einem Dünnschichtverdampfer in großem Maßstab, von unerwünschten Nebenprodukten, Reaktionsedukten oder Abbauprodukten befreit werden, allerdings bleiben mit den derzeitigen Herstellungsverfahren hohe Kosten, geringe Selektivitäten und eine geringe Materialeffizienz verbunden.

Thermische Umlagerungsreaktion bilden oft die Grundlage vieler synthetischer Verfahren und finden oft nur unter harschen thermischen Bedingungen statt. Allerdings führen derartig hohe thermische Belastungen oft zur thermischen Zersetzung der daraus hervorgehenden Verbindungen oder von entsprechenden Nebenprodukten, welche nachteilig mit den Produkten wechselwirken können. Dadurch ist keine ausreichende Selektivität der Herstellung und somit keine gute Qualität der Produkte mehr gewährleistet. So erfolgt beispielsweise die Synthesen von 3-(4-Isopropylcyclohexen-1-yl)propanal, wie bereits in EP 2578671 A1 beschrieben, über eine thermisch getriebene Claisen-Umlagerung. Allerdings ist dabei eine geringe Selektivität der der Reaktion zu beobachten, da bei der sauren Spaltung des Acetals ebenfalls das Nebenprodukt 5-Isopropyl-2-methylen-cyclohexanol gebildet wird.

Dokument EP 2247648 B1 beispielsweise beschreibt ein Verfahren zur Herstellung von Lactamaten durch Dünnschichtverdampfung durch Umsetzung von Alkoholaten mit Lactamen. Dabei findet auf dem Dünnschichtverdampfer eine Reaktion des Alkoholats mit dem Lactam unter Ausbildung des katalytisch-wirkenden Lactamats statt. Bei dieser Reaktion wird der korrespondierende Alkohol freigesetzt, der sodann unmittelbar auf dem Dünnschichtverdampfer der Reaktionsmischung entzogen wird.

Patentschrift WO 2005030358 A1 offenbart einen Dünnschichterdampfer ausgestaltet zur Erhöhung der Trennleistung und gegebenenfalls zur Durchführung oder Beschleunigung chemischer Reaktionen während des Verdampfungsprozesses. Hierzu ist in einer bevorzugten Ausführungsform der Innenraum des Dünnschichtverdampfers als Katalysator ausgebildet. Versuche zeigten, dass sich eine Kombination von Destillation, Absorption und chemischer Reaktion durch den Einbau heterogener Katalysatoren in den Verdampfungsraum und/oder durch den Einbau von Stoffaustauschflächen sowie durch eine Zugabe von Reaktionspartnern direkt in den Verdampfungsraum durchführen ließ.

In US 5561209 A ist ein kontinuierliches Verfahren zur Herstellung von Polyorganosiloxanen durch Kondensationsreaktion von niedermolekularen Polyorganosiloxanolen untereinander oder mit oligomeren Siloxanen in Gegenwart eines Katalysatorsystems in einem Dünnschichtverdampfer offenbart.

Ein kontinuierliches Verfahren zur Herstellung des Riechstoffs Citral wird in WO 2008037693 A1 offenbart. Insbesondere wird darin auf die Herstellung der entsprechenden Acetale eingegangen. Die darin beschriebene Apparatur zur Herstellung der ungesättigten Acetale besteht vorzugsweise aus einer Destillationskolonne, die als Reaktionskolonne benutzt wird, wobei das entstandene Citral schon während der Umsetzung fortlaufend destillativ aus dem Reaktionsgemisch entfernt wird.

Ein alternativer kontinuierlicher Prozess für die Herstellung on Citral durch thermische Spaltung von 3-Methyl-2-buten-1-al-diprenylacetal wird in EP 0992477 B1 beschrieben. Dabei wir die thermische Spaltung des Acetals im unteren Teil oder im Sumpf einer Destillationskolonne mit 5 bis 100 theoretischen Trennstufen durchführt.

Keines der zitierten Dokumente des Standes der Technik beschreibt jedoch die Herstellung von Riechstoffen oder Duftstoffen unter Durchführung von thermischen Umlagerungsreaktionen unter kontrollierten und materialeffizienten Gesichtspunkten in einem Dünnschichtverdampfer. Die hohe Effektivität des darin beschriebenen Prozesses ist auf milde Reaktionsbedingungen zurückzuführen, wodurch thermische Belastungen der Produkte und Nebenreaktionen vermieden werden.

Der Erfindung liegt somit die allgemeine Aufgabe zu Grunde ein Verfahren bereitzustellen, bei dem Riechstoffe oder Duftstoffe effizient und schonend hergestellt werden können.

Eine weitere Aufgabe der Erfindung bezieht sich auf ein hochselektives Verfahren zur Herstellung von 3-(4-Isopropylcyclohexen-1-yl)propanal.

Daher betrifft die vorliegende Erfindung ein effizientes Herstellungsverfahren in Kombination mit effektiver Aufreinigung der hergestellten Riechstoffe oder Duftstoffe in einem einzelnen kombinierten Verfahrensschritt.

Unter einem weiteren Gesichtspunkt betrifft die vorliegende Erfindung auch die material- und kosteneffiziente Herstellung von Riech- oder Duftstoffen unter Berücksichtigung von Umweltaspekten.

### Zusammenfassung der Erfindung

Die vorliegende Problemstellung wird durch die Gegenstände der unabhängigen Patentansprüche gelöst. Bevorzugte Ausgestaltungen ergeben sich aus dem Wortlaut der abhängigen Patentansprüche sowie der nachfolgenden Beschreibung sowie den Ausführungsbeispielen.

Die vorliegende Erfindung betrifft in einem ersten Aspekt ein Verfahren zur Herstellung eines Riechstoffs oder Duftstoffs unter Durchführung von thermischen Umlagerungsreaktionen umfassend die folgenden Schritte:
a) Bereitstellen von mindestens einem Reaktionsedukt und/oder weiteren Reagenzien in einem Dünnschichtverdampfer;
b) Ausführen mindestens einer thermischen Umlagerungsreaktion an den Reaktionsedukten aus Schritt a) im Dünnschichtverdampfer unter destillativen Bedingungen;
c) Erhalt des Riechstoffs oder Duftstoffs.

In einer nächsten bevorzugten Variante des zuvor beschriebenen Verfahrens, ist das Produkt aus Schritt c) die Verbindung 3-(4-Isopropylcyclohexen-1-yl)propanal. Somit betrifft eine bevorzugte Weiterbildung der vorliegenden die Erfindung ein Verfahren zur Durchführung von thermischen Umlagerungsreaktionen im Dünnschichtverdampfer unter destillativen Bedingungen für die Herstellung des Riechstoffs 3-(4-Isopropylcyclohexen-1-yl)propanal.

Überraschend wurde im Rahmen der vorliegenden Erfindung gefunden, dass bei der Durchführung von thermischen Umlagerungsreaktionen in einem Dünnschichtverdampfer die Selektivität von thermischen Umlagerungsreaktionen erhöht werden kann, was wiederum aufgrund der milderen Reaktionsbedingungen in höheren Ausbeuten und einer gesteigerten Reinheit der chemischen Produkte resultiert. Es ist bekannt, dass bei höheren Temperaturen, d. h. höherer thermischer Energie, die Selektivität von chemischen Reaktionen sinkt. Im Umkehrschluss bedeutet dies, dass bei niedrigen Temperaturen, also geringerer thermischer Energie, die Selektivität steigt, wohingegen bei hohen Temperaturen eine nahezu gleichmäßige Produktverteilung zu erwarten ist. Allerdings ist hierbei zu berücksichtigen, dass oft auch mit geringeren Temperaturen chemische Reaktionen langsamer verlaufen können, was unter Umständen in längeren Produktionszeiten resultieren kann. Es hat sich gezeigt, dass das Verfahren entsprechend der vorliegenden Erfindung eine optimierte Abstimmung zwischen milden Reaktionsbedingungen und effizienter Umsetzung der Reaktionsedukte darstellt und daher einen optimalen Kompromiss zwischen Ausbeute, Reinheit und Produktivität bietet.

Die milden Bedingungen der vorliegenden Erfindung beziehen sich allerdings nicht nur auf die Temperaturen als solche, sondern auch auf die Verweilzeiten des Reaktionsgemisches an der beheizen Verdampferinnenfläche. Das erfindungsgemäße Verfahren ermöglicht deutlich verkürzte lokale Verweilzeiten und reduziert somit die Gefahr lokaler thermischer Belastungen, insbesondere im Hinblick bereits umgesetzter, d. h. umgelagerter Reaktionsteilnehmer und reduziert somit thermische Zersetzungen, d. h. thermische Produktschädigung. Daher eignet sich das vorgestellte Verfahren besonders im Zusammenhang mit wärmeempfindlichen/temperatursensitiven Riech- und Duftstoffen.

Diese und weitere Aspekte, Merkmale und Vorteile der vorliegenden Erfindung werden für den Fachmann aus dem Studium der folgenden detaillierten Beschreibung und der Patentansprüche ersichtlich. Dabei kann jedes Merkmal aus einem Aspekt der Erfindung in einem anderen Aspekt der Erfindung eingesetzt oder ausgetauscht werden. Die in der vorliegenden Anmeldung enthaltenen Beispiele beschreiben die Erfindung, ohne diese einzuschränken.

Numerische Beispiele, die in der Form "von x bis y" angegeben sind, schließen die genannten Werte ein. Wenn mehrere bevorzugte numerische Bereiche in diesem Format angegeben sind, sind alle Bereiche, die durch die Kombination der verschiedenen Endpunkte entstehen, ebenfalls erfasst werden.

Vorteilhafte Weiterbildungen und Varianten der Erfindung sind in den abhängigen Ansprüchen angegeben.

### Detaillierte Beschreibung der Erfindung

Ein erster Gegenstand der vorliegenden Erfindung betrifft ein Verfahren zur Herstellung eines Riechstoffs oder Duftstoffs unter Durchführung von thermischen Umlagerungsreaktionen umfassend die folgenden Schritte:
a) Bereitstellen von mindestens einem Reaktionsedukt und/oder weiteren Reagenzien in einem Dünnschichtverdampfer;
b) Ausführen mindestens einer thermischen Umlagerungsreaktion an den Reaktionsedukten aus Schritt a) im Dünnschichtverdampfer unter destillativen Bedingungen;
c) Erhalt des Riechstoffs oder Duftstoffs.

In dem Verfahren zur Durchführung von thermischen Umlagerungsreaktionen wird in einem ersten Schritt a) mindestens ein Reaktionsedukt bereitgestellt.

In Abhängigkeit davon, ob die besagte thermische Umlagerungsreaktion in Gegenwart weiterer Reagenzien, wie beispielsweise von Katalysatoren, stattfindet, werden auch weitere entsprechende Reagenzien in Schritt a) des erfindungsgemäßen Verfahrens bereitgestellt.

Eine bevorzugte Ausführungsform der vorliegenden Erfindung, betrifft thermische Umlagerungsreaktionen, welche saure Bedingungen erfordern und/oder unter Säure-Katalyse erfolgen. In dem genannten Fall umfasst Schritt a) der vorliegenden Erfindung das Bereitstellen von mindestens einem Reaktionsedukt und/oder mindestens einer Säure in einem Dünnschichtverdampfer.

Geeignete Säuren in diesem Kontext sind beispielsweise organische Säuren und deren saure Salze, wie z. B. Carbonsäuren, Alkohole, Phenole, Enole, Thiole, Schwefelsäureester und Sulfonsäuren, Phosphorsäureester und Phosphonsäure, CH- und NH-acide Verbindungen und deren Salze. Weiterhin geeignet sind auch anorganische Säuren sowie deren saure Salze, wie z. B. Salzsäure, Kohlensäure, Phosphorsäure, Salpetersäure, Salpetrige Säure, Schwefelsäure, Schweflige Säure, Thioschwefelsäure und deren Salze. Besonders bevorzugt idt jedoch vorliegend die Verwendung von organischen Säuren und deren Salzen in Schritt a).

Vorzugsweise finden aliphatische Carbonsäuren, substituierte Carbonsäuren, heterocyclische Carbonsäuren sowie aromatische Carbonsäuren und deren Salze (Carboxylate) im Rahmen des hierin beschriebenen Verfahrens Anwendung. Auch Verbindungen, in denen die OH-Gruppe der Carboxygruppe durch eine andere Gruppe, z. B. -OR, -NH₂ oder -Cl ersetzt ist (Carbonsäurederivate) wie beispielsweise Carbonsäureester, Carbonsäureamide und Carbonsäurehalogenide und deren Salze sind geeignete Säuren im Kontext der vorliegenden Erfindung.

Diese weisen dabei vorzugweise eine oder mehrere Carboxygruppen (-COOH) auf. Geeignete Carbonsäuren sind unter anderem: Essigsäure, Acrylsäure, Oxalsäure, Ameisensäure, Trifluoressigsäure, Bernsteinsäure, Fumarsäure, Maleinsäure, Trichloressigsäure, Zitronensäure, aromatische Carbonsäuren wie z. B. Acetylsalicylsäure, Benzoesäure, Phenylessigsäure und Salicylsäure, Aminosäuren wie z.B. Alanin, Asparaginsäure und Glycin, sowie die Salze der zuvor genannten Säuren.

In diesem Zusammenhang besonders bevorzugte Säuren sind ausgewählt aus der Gruppe, die besteht aus: organischen Säuren und deren sauren Salzen, wobei insbesondere Carbonsäuren mit einer oder mehreren Carboxygruppen zu bevorzugen sind sowie die sauren Salze der zuvor genannten Verbindungen.]

In einer weiteren bevorzugten Ausgestaltung der vorliegenden Erfindung ist die Verwendung sowie die Bereitstellung von Salicylsäure in Schritt a) besonders bevorzugt.

Unter dem Begriff "Reaktionsedukt" wird verstanden, dass die in dem erfindungsgemäßen Verfahren zum Einsatz kommenden Reaktanden den Ausgangsstoff der thermisch bedingten Umlagerungsreaktion darstellen. Diese selbst können jedoch aus weiteren Edukten in weiteren vorangehenden Schritten hergestellt worden sein.

Der erfindungsgemäße Schritt b) des hierin beschriebenen Verfahrens umfasst das Ausführen mindestens einer thermischen Umlagerungsreaktion an den Reaktionsedukten aus Schritt a) im Dünnschichtverdampfer unter destillativen Bedingungen.

Thermische Umlagerungsreaktionen betreffen all jene chemische Reaktionen, bei denen durch Verschiebungen einzelner Atome oder Atomgruppen einhergehend mit Bindungsspaltungen und -neubildungen innerhalb eines Moleküls, d. h. intramolekular, strukturell betrachtet neue chemische Verbindungen gebildet werden. Thermische Umlagerungen werden mittels thermischer Energie initiiert, d. h. thermische Energie in Form von Wärme muss zugeführt werden, damit die thermische Umlagerungsreaktion stattfindet.

Bei den hierin beschriebenen Reaktionsedukten handelt es sich deshalb vorzugsweise um chemische Verbindungen, welche nach Durchführung von thermischen Umlagerungsreaktionen, interessante und überraschende Eigenschaften aufweisen. Solche Produkte können demzufolge vorzugsweise Riech- oder Duft- und Aromastoffe sein, wie beispielsweise die Verbindung 3-(4-Isopropylcyclohexen-1-yl)propanal der Formel (I), welche als Maiglöckchen-Riechstoff Verwendung findet. Daher betrifft die vorliegende Erfindung in einer bevorzugten Ausgestaltung ein Verfahren zur Durchführung von thermischen Umlagerungsreaktionen für die alternative Herstellung von Riech- und Aromastoffen, vorzugsweise des Riechstoffs 3-(4-Isopropylcyclohexen-1-yl)propanal.

Grundsätzlich sind jedoch all jene Verbindungen als Rektionsedukte denkbar, welche thermische Umlagerungsreaktionen durchlaufen können.

Sind mehrere Reaktionsedukte oder Reagenzien an der Umlagerung beteiligt, so ist es von Vorteil diese in homogenisierter Art und Weise dem Dünnschichtverdampfer zuzuführen.

Zu diesem Zeitpunkt, d. h. im Rahmen von Verfahrensschritt a), findet üblicherweise noch keine Umsetzung statt, da die für den Ablauf der thermischen Umlagerung notwendige thermische Aktivierungsenergie erst noch der Reaktionseduktmischung zugeführt werden muss. Dies geschieht vorzugsweise nach Einspeisung in den Dünnschichtverdampfer, erst im Inneren des Dünnschichtverdampfers.

Unter einem Dünnschichtverdampfer wird im Rahmen der vorliegenden Erfindung eine Apparatur zur destillativen Auftrennung von Reaktionsgemischen verstanden, worin das aufzutrennende Reaktionsgemisch mittels speziell konstruierter Wischer gleichmäßig und in einem definierten dünnen Film auf den beheizten Verdampferinnenflächen verteilt wird. Besonders bevorzugt sind hierin vertikal konstruierte Dünnschichtverdampfer, ausgestaltet mit einer innenliegenden Vorrichtung zur Verteilung der Reaktionsmischung sowie einer außenliegenden Vorrichtung zur Beheizung der Verdampferinnenflächen. Die vorbereitete Reaktionsmischung umfassend das mindestens eine Reaktionsedukt und/oder weitere Reagenzien wird vorzugsweise im oberen Teil des Dünnschichtverdampfers eingespeist und anschließend mittels rotierender Wischelemente als dünner Film auf die beheizte Verdampferinnenfläche verteilt.

Daher ist der im Rahmen des vorliegenden Verfahrens verwendete Dünnschichtverdampfer vorzugsweise mit einem rotierenden Wischersystem ausgestattet. Die Wahl des entsprechenden Wischsystems hängt dabei von den Eigenschaften der Reaktionsmischung, wie beispielsweise ihrer Viskosität, ab.

Der hierin verwendete Dünnschichtverdampfer weist einen zylindrischen Innenraum auf, dessen Verdampferinnenflächen auf eine definierte Temperatur aufgeheizt werden können, eine Dosierpumpe am oberen Teil der Rührblätter ausgestaltet zum Einspeisen der Reaktionsmischung, ein rotierendes Wischsystem mit justierbaren Umdrehungsgeschwindigkeiten sowie eine Vakuumpumpe zur Einstellung des Drucks, einem Auffangglaskolben am unteren Ende sowie eine Kühlung und Kopfabnahme für die Brüden auf.

In der vorliegenden Erfindung findet daher auf dem Dünnschichtverdampfer, d. h. auf den Verdampferinnenflächen des Dünnschichtverdampfers, eine thermische Umlagerung des mindestens einem Reaktionsedukt und/oder weiteren Reagenzien statt. Bei einer derartigen Reaktion werden Reaktionsnebenprodukte und oder nicht umgesetzte Reaktionsedukte mittels Destillation unmittelbar in dem Dünnschichtverdampfer der Reaktionsmischung entzogen.

Die Formulierung "unter destillativen Bedingungen" bezieht sich daher auf die Abtrennung von gewissen Reaktionsteilnehmern (Reaktionsprodukte oder aber - edukte) aus dem Reaktionsgemisch durch thermische Verdampfung und anschließende Wiederverflüssigung im klassischen Sinne. Stofftrennungen durch Verdampfung und anschließende Kondensation zählen somit zu den thermischen Trennverfahren.

Die gleichzeitige Abtrennung von Reaktionsnebenprodukten und Reaktionsprodukten, welche im Sumpf innerhalb des Auffangkolbens angereichert werden, ermöglicht eine kontinuierliche Verschiebung des Reaktionsgleichgewichtes auf die Produktseite, was eine möglichst vollständige Umsetzung der Reaktionsedukte ermöglicht.

Derartige Verfahren können im Allgemeinen daher in die Klasse der Reaktivdestillationen eingeordnet werden, da hierbei eine Reaktion, hier die thermische Umlagerung, und ein Trennprozess, nämlich die destillative Abtrennung der bei der Umlagerung gebildeten Nebenprodukte, unter einem Verfahrensschritt zusammengefasst werden. Das erfindungsgemäß vorgesehene Verfahren umfassend eine Reaktivdestillation wird daher auf einem Dünnschichtverdampfer durchgeführt.

Die Leichtsieder bestehend aus Reaktionsnebenprodukten und/oder nichtumgesetzten Reaktionsedukten werden somit am Kopf der Kolonne abdestilliert und anschließend einkondensiert, während das Produkt im Sumpf innerhalb des Auffangkolbens angereichert wird. Dies führt unter anderem zu einer Aufkonzentrierung des Produktes. Die Abdestillierten Verbindungen können in weiteren fein-destillativen Schritten weiter aufgereinigt werden und stehen anschließend einer Wiederverwendung als Reaktionsedukte oder Reagenzien in anderen Herstellungsverfahren zu Verfügung.

In einer weiteren bevorzugten Ausgestaltung des vorliegenden Verfahrens findet die thermische Umlagerungsreaktion unter destillativen Bedingungen unter zusätzlichen inerten Bedingungen statt.

In einer alternativen Ausführungsform hiervon kann ein zusätzlicher Strippvorgang vorgesehen sein.

Die Verwendung von Dünnschichtverdampfern als Reaktionsgefäß für die Herstellung von Riech- oder Duftstoffen bietet die Möglichkeit, die darin enthaltenen Reaktionsmischungen in Form eines dünnen Films gleichmäßig zu verdampfen. Da der Dünnschichtverdampfer in der vorliegenden Erfindung vorzugsweise im Vakuum betrieben wird, ermöglicht das hierin beschriebene Verfahren die Verwendung geringerer Temperaturen und eignet sich deshalb zur schonenden thermischen Umlagerung und gleichzeitigen Trennung von Nebenprodukten und ermöglicht es somit die Umsetzung der Edukte als auch die Abtrennung und Aufreinigung der Produkte in einem einzelnen Verfahrensschritt gleichzeitig zu vereinen.

Weiterhin sorgen die ständig rotierenden Wischerblätter des Dünnschichtverdampfers für eine gleichmäßige Verteilung der Reaktionsedukte, sodass beim Durchfluss durch das Innere des Dünnschichtverdampfers bereits die vollständige Einstellung des Reaktionsgleichgewichtes erfolgt. Weiterhin werden kürzere Verweildauern der Reaktionsteilnehmer an den Heizflächen und gleichzeitig hohe Verdampfungsraten erzielt, wodurch sich das hierin beschriebenen Verfahren insbesondere für die Umsetzung temperaturempfindlicher Reaktionsedukte eignet. Diese reduzierten thermischen Belastungen der verwendeten Reaktionsedukte, aber auch der Reaktionsprodukte, insbesondere aufgrund der verkürzten Verweilzeiten und vermiedenen lokalen Überhitzungen der Reaktionsteilnehmer gegenüber gängigen Kolben-Versuchen, zeichnen das erfindungsgemäße Verfahren als ein sehr schonendes Verfahren aus, welches ungewollte thermische Zersetzungen reduziert.

Die gute Mischwirkung in Zusammenhang mit der geringen Dicke des Films ergeben maximale Verdampfungsraten und minimierte Verweilzeiten im Verdampfer bei moderaten Temperaturen, wodurch die Herstellung quantitativ und qualitativ hochwertiger (temperaturempfindlicher) Riech- und Duftstoffe ermöglicht wird.

Die Betriebseinstellungen des Dünnschichtverdampfers, insbesondere die Wahl von Temperatur, Druck, Flüssigkeitsdurchsatz des Reaktionsgemisches und Wischgeschwindigkeit, d. h. die Rotationsgeschwindigkeit des Wischersystems, hängen maßgeblich von der Art der thermischen Umlagerungsreaktion sowie der eingesetzten Reaktionsedukte ab und werden entsprechend an diese angepasst, um die die typischen Verweilzeiten der chemischen Komponenten an der Verdampfungsfläche, d. h. an der Heizfläche zu minimieren und eine schonende Umsetzung als auch Trennung zu gewährleisten.

Eine schonender Umsetzung beziehungsweise Trennung bedeutet in der vorliegenden Erfindung somit, dass die thermische Belastung und die Verweilzeit während der Umlagerung und/oder Destillation gering sind und das entsprechende Reaktionsprodukt dabei keine negative thermische Schädigung erleidet, die sich in einer thermischen Zersetzung oder einer Veränderung der Produkteigenschaften wie Farbe, Geruch, Stabilität etc. bemerkbar machen kann.

Die somit gewährleisteten schnellen Reaktionen und damit verbundenen kurze Verweilzeiten ermöglichen eine Steigerung der Raum-Zeit Ausbeute und reduzieren gleichzeitig die thermische Belastung der Reaktionsteilnehmer. Eine schnelle Abtrennung des gebildeten Reaktionsproduktes aus der Reaktionsmischung ermöglicht höhere Ausbeuten. Weiterhin zeichnet sich das hierin beschriebene Verfahren durch seine geringe Komplexität im Vergleich zu mehrstufigen Verfahren aus. Somit zeichnet ist das hierin beschriebene Verfahren als ein technisch sowie ökonomisch vorteilhaftes Verfahren aus, welches sich insbesondere aufgrund der hohen Raum-Zeit-Ausbeute und Einfachheit der Durchführung als großtechnisches Verfahren eignet.

Ein weiterer Vorteil des hierin beschriebenen Verfahrens liegt darin, dass thermische Umlagerungsreaktionen, welche hohe Temperaturen zur Aktivierung des Umlagerungsprozesses benötigen, durch Anlegen eines Vakuums an den Dünnschichtverdampfer unter Verwendung moderater Temperaturen durch Anlegen eines entsprechenden Vakuums an den Dünnschichtverdampfer realisiert werden können, was sich positiv auf die Energiekosten auswirkt und eine thermische Zersetzungen verringert.

Insgesamt hat sich also überraschend gezeigt, dass bei der Durchführung von thermischen Umlagerungsreaktionen in einem Dünnschichtverdampfer gemäß dem erfindungsgemäßen Verfahren die Selektivität von thermischen Umlagerungsreaktionen erhöht wird, und höhere Ausbeuten und einer gesteigerte Reinheit der chemischen Produkte erzielt werden können.

Weiterhin zu betonen ist der geringe apparative Aufwand einhergehend mit dem beschriebenen Verfahren. Insbesondere im Hinblick auf den Dünnschichtverdampfer, welcher sowohl als Reaktor für die thermische Umlagerung als auch gleichzeitig als Destillationsapparatur fungiert und somit spezifische Apparaturen für die Durchführung der thermischen Umlagerungsreaktion einerseits und einer gesonderten Destillationsvorrichtung für die Aufreinigung andererseits unnötig macht.

Dadurch entfallen mühselige Umbauten und Überführungen des Reaktionsproduktes, was mit Verlusten der Ausbeute verbunden wäre. Zudem ist hierin der zeitliche Aspekt zu betrachten. Zeitliche und apparative Einsparungen spiegeln sich daher in verringerten Produktionskosten wider. Das vorliegende Verfahren eignet somit hervorragend für die großtechnische Herstellung chemischer Produkte über thermische Umlagerungen.

Die Reaktivdestillation wird vorzugsweise bei einer Betriebstemperatur des Dünnschichtverdampfers von vorzugsweise 160 bis 260 °C, bevorzugt von 180 bis 240 °C und weiter bevorzugt bei einer Betriebstemperatur von 230 °C, durchgeführt. Dies entspricht einer ungefähren Reaktionstemperatur von 220 °C im Inneren des Dünnschichtverdampfers.

Die tatsächliche Kontakttemperatur der Reaktanden hingegen ist vorzugsweise 160 bis 240 °C, noch bevorzugter von 180 bis 220 °C. Besonders bevorzugt ist die Reaktionstemperatur bzw. Kontakttemperatur 220 °C.

Vorzugsweise beträgt die Verweilzeit der Reaktanden an der Dünnschichtverdampferinnenfläche nur wenige Sekunden, und ist somit deutlich kürzer als die bei einer Reaktivdestillation.

Der Druck im Verdampfer beträgt vorzugsweise 500 bis 1000 mbar, besonders bevorzugt ca. 200 mbar, wobei der zu verwendende Druck jeweils von dem herzustellenden Produkt abhängt.

Das Reaktionsprodukt der thermischen Umlagerungsreaktion in Schritt c) des hierin beschriebenen Verfahrens wird dabei vorzugsweise am unteren Ende des Dünnschichtverdampfers in einem Auffangkolben erhalten. Das dort angereicherte Produkt wird dabei in hoher Ausbeute und hoher Reinheit basierend auf der hohen Selektivität des hierin beschriebenen Verfahrens erhalten.

Die Verbindung 3-(4-Isopropylcyclohexen-1-yl)propanal findet vorrangig Verwendung als Riechstoff mit Maiglöckchennote. Trotz einer Vielzahl bereits vorhandener Riechstoffe besteht in der Parfümindustrie auch weiterhin ein genereller Bedarf an neuen Riechstoffen, welche eine unverzichtbare Komponente in der Riechstoffindustrie sowie bei der Herstellung von Kosmetika, Körperpflegemitteln sowie Wasch- und Reinigungsmitteln etc. sind. Neben der Suche nach neuen Riechstoffen, stellt die Optimierung der Herstellungsverfahren hinsichtlich Effizienz, Ressourcenschonung und umwelttechnischer Aspekte derzeit eine primäre Herausforderung dar, um der hohen Nachfrage entgegenzukommen und hochwertige Produkte und Inhaltsstoffe anbieten zu können.

Derzeit verwendete Herstellungsverfahren des Riechstoffs 3-(4-Isopropylcyclohexen-1-yl)propanal umfassend eine thermische Umlagerung des entsprechenden Acetals unter Spaltung eines Vinylethers zu dem gewünschten Produkt. Allerdings zeigen die gängigen Herstellungsverfahren geringe Selektivitäten und liefern daher nur geringe Ausbeuten und geringe Reinheit. Darüber hinaus eignen sich die zuvor beschrieben Verfahren nicht für eine materialeffiziente und kostensenkende Herstellung.

In einer bevorzugten Ausgestaltung der vorliegenden Erfindung betrifft das hierin beschriebene Verfahren ein optimiertes, alternatives Verfahren zur Herstellen 3-(4-Isopropylcyclohexen-1-yl)propanal.

Weiterhin bevorzugt ist die Verwendung des erfindungsgemäßen Verfahrens als alternative Herstellungsmethode für die Bereitstellung des beliebten Riech- und Aromastoffs 3,7-Dimethylocta-2,6-dienal, auch bekannt unter der Bezeichnung Citral.

Ein Riechstoff oder Duftstoff ist im Rahmen des vorliegenden Textes jede Substanz, die dazu geeignet ist, zum Hervorrufen eines geruchlichen Eindrucks verwendet zu werden, d. h. zum Vermitteln eines geruchlichen Eindrucks, oder zum Verändern (Modifizieren oder Verstärken) der geruchlichen Wahrnehmung einer anderen Substanz. Dabei soll diese Substanz, damit sie für parfümistische Zwecke eingesetzt werden kann, vorzugsweise keine unerwünschten Nebenwirkungen, z. B. die Gesundheit oder die Umwelt schädigende Wirkungen, oder den bestimmungsgemäßen Gebrauch eines Produkts, das diesen Riech- oder Duftstoff enthält, beeinträchtigende Wirkungen haben darf.

Wie bereits zu Beginn erwähnt, weisen Riechstoffe oft sehr spezifische Geruchsprofile auf. Daher ist es besonders wünschenswert derartige Produkte mit hoher Reinheit, d. h. von Verunreinigungen und möglichst selektiv zu synthetisieren, da bereits geringe Mengen an Verunreinigungen den charakteristischen Geruchseindruck verfälschen oder nachteilig beeinflussen können und unangenehme Nebengerüche verursachen können. Derartige Verunreinigungen können sich ferner aufgrund von chemischen Wechselwirkungen negativ auf die Stabilität der hergestellten Riechstoffe auswirken und unangenehme Geruchsnoten hervorrufen oder den zugrundeliegenden Geruchseindruck verändern. Daher ist ein möglichst hoher Reinheitsgrad solcher Verbindungen gewünscht.

Gegenwärtige Herstellungsverfahren erzeugen oftmals Riechstoffe in nur unzureichender Reinheit und bedürfen daher weiterer Optimierung. Darüber hinaus sind aufgrund der hohen Nachfrage nach Riech- oder Duftstoffen zeit-, material- und daher kosteneffizientere Verfahren gefragt, um die derzeit noch aufwendigen und teuren und daher unwirtschaftlichen Herstellungswege zu ersetzen.

Zwar können beispielsweise Riech- und Duftstoffe derzeit wie bereits oben erwähnt mittels Destillation, z. B. in einem Dünnschichtverdampfer in großem Maßstab, von unerwünschten Nebenprodukten, Reaktionsedukten oder Abbauprodukten befreit werden, allerdings bleiben mit den derzeitigen Herstellungsverfahren hohe Kosten, geringe Selektivitäten und eine geringe Materialeffizienz verbunden.

Es hat sich überraschend gezeigt, dass sich ein erfindungsgemäßes Verfahren für die selektive und somit hoch reine Synthese von Riech- oder Duftstoffen eignet. Die synthetisierten Riech- oder Duftstoffe weisen daher ein stabiles Geruchsprofil ohne Nebengerüche auf und eignen sich somit für die Weiterverarbeitung in parfümierten Produkten oder zu Parfümölen.

Das erfindungsgemäße Verfahren eignet sich daher insbesondere für die wirtschaftliche Herstellung hoch konzentrierter und reiner Riech- oder Duftstoffe in hohen Ausbeuten durch die Kombination aus schonender und selektiver Synthese mit reduzierter thermischer Belastung und gleichzeitiger Destillation und somit Aufreinigung der Produkte.

Eine weitere bevorzugte Weiterbildung der vorliegenden Erfindung betrifft ein Verfahren nach dem ersten Erfindungsgegenstand, wobei die mindestens eine thermischen Umlagerungsreaktion mindestens eine pericyclische Reaktion umfasst.

In diesem Zusammenhang sind unter pericyclischen Reaktionen Umlagerungsreaktionen zu verstehen, welche durch eine konzertierte, d. h. gleichzeitige intramolekulare Verschiebung der Elektronen einen cyclischen Übergangszustand durchlaufen, ohne dass dabei radikalische oder ionische Zwischenstufen auftreten. Beispiele derartiger Reaktionen sind beispielsweise sigmatrope Umlagerungen, insbesondere des Claisen oder Cope-Typs, Cycloadditionen, chelatrope Reaktionen oder elektrocyclische Reaktionen. Grundsätzlich können pericyclische Umlagerungsreaktionen entweder thermisch oder photochemisch initiiert ablaufen, wobei in der vorliegenden Erfindung thermisch getriebene Umlagerungsreaktionen bevorzugt sind.

Es hat sich gezeigt, dass derartige Umlagerungen besonders selektiv nach dem vorliegenden Verfahren durchführbar sind, was sich in einer hohen Ausbeute und hoher Reinheit der Produkte widerspiegelt. Daher eignet sich das vorliegende Verfahren besonders für die Durchführung pericyclischer Umlagerungen im Zusammenhang mit der Herstellung Riech- oder Duftstoffen.

Wie zuvor im Hinblick des ersten Erfindungsgegenstandes erläutert, resultiert hierbei die Selektivität der thermischen Umlagerungsreaktionen insbesondere aus den milden Verfahrensbedingungen.

Eine alternative Variante des ersten Erfindungsgegenstandes betrifft ein Verfahren, betreffend die Ausführung von mindestens einer Umlagerung des Typs der Claisen-Umlagerung, der Cope-Umlagerung und/oder der intramolekularen Prins-Reaktion.

Bezüglich sigmatropen Umlagerungsreaktionen sind hierbei insbesondere Claisen-Umlagerungen und Cope-Umlagerungen interessant. Diese Reaktionen finden allein oder in Kombination unter anderem Anwendung in der Synthese bekannter Riechstoffverbindungen wie beispielsweise Citral, ein Gemisch aus den cistrans-Isomeren Geranial und Neral.

In einer bekannten Synthese des Riechstoffs findet zunächst eine Spaltung des entsprechenden Acetals und anschließende Umlagerungsreaktion nach Claisen und Cope statt. In EP 0992477 B1 wird ein fortlaufender destillativen Prozess für die Synthese von Citral beschrieben. Entsprechend WO 2008037693 A1 erfolgt sie Synthese in einer Destillationskolonne, die als Reaktionskolonne benutzt wird, wobei die Dämpfe gasförmig in die Kolonne zurückgeleitet werden.

Es hat sich überraschend gezeigt, dass derartige Reaktionen und Synthesen in einem Dünnschichtverdampfer effizient durchgeführt werden können. Dabei werden die Reaktionsedukte nicht im Sumpf der Kolonne umgesetzt, sondern in einem feinen Film entlang der Verdampferinnenfläche des Dünnschichtverdampfers. Im Gegensatz zu den zuvor beschriebenen Kolben-Versuchen werden durch ständige Umwälzung des Reaktionsgemisches durch das Wischersystem lokale Überhitzungen vermieden und die thermische Belastung der Reaktanden weitestgehend reduziert.

Die säurekatalysierte Carbonyl-En-Reaktion, auch Prins-Reaktion genannt, zählt zur Gruppe der Cycloadditionen. Anwendung findet eine intramolekulare Prins-Reaktion, d. h. intramolekulare Carbonyl-En-Reaktion, beispielsweise in der Synthese des Riechstoffes Isopulegol ausgehend von Citronellal.
Überraschend wurde festgestellt, dass auch der Riechstoff Isopulegol in hoher Ausbeute und hoher Reinheit in mittels des hierin beschriebenen Verfahrens basierend auf der Durchführung von thermischen Umlagerungsreaktionen in einem Dünnschichtverdampfer unter destillativen Bedingungen hergestellt werden konnte.

Wie hieraus ersichtlich wird, lässt sich die Herstellung einer Vielzahl von Riech- oder Duftstoffen auf pericyclische Umlagerungsreaktionen des Claisen-, des Cope- und des Prins-Typs zurückführen.

Es hat sich überraschend gezeigt, dass diese Umlagerungen einzeln oder in Kombination besonders selektiv nach dem vorliegenden Verfahren ablaufen. Die daraus resultierenden Produkte weisen eine hohe Ausbeute und hohe Reinheit auf. Daher eignet sich das vorliegende Verfahren besonders für die Durchführung mindestens einer Umlagerung nach Claisen, nach Cope und/oder Prins einzeln oder in Kombinationen daraus, insbesondere für die Herstellung von Riech- oder Duftstoffen.

In einer bevorzugten Ausgestaltung der vorliegenden Erfindung, betrifft das erfindungsgemäße Verfahren zur Durchführung von thermischen Umlagerungsreaktionen die Durchführung von Umlagerungen nach Claisen.

In einer bevorzugten Ausgestaltung der vorliegenden Erfindung, betrifft das erfindungsgemäße Verfahren zur Durchführung von thermischen Umlagerungsreaktionen die Durchführung von Umlagerungen nach Cope.

Sigmatrope Umlagerungen sind eine besondere Form der pericyclischen Reaktionen und zeichnen sich durch die Verschiebung von σ-Bindungen aus. Dabei bleibt die Anzahl an σ- und π-Bindungen vor und nach der Umlagerung gleich.

Oxy-Cope-Umlagerungen benötigen eine geringere Aktivierungsenergie als Cope-Umlagerungen und finden daher bereits bei geringeren Temperaturen statt. Derartige Claisen-Reaktionen (Oxy-Cope-Umlagerungen) sind daher ebenso wie die bekannte Cope-Umlagerung [3,3]-sigmatrope Umlagerung, bei denen ein Kohlenstoffatom durch ein Sauerstoffatom ersetzt ist.

Nacheinander stattfindende Umlagerungsreaktionen, sogenannte Tandem-Reaktionen, sind intramolekulare Reaktionsfolgen von Umlagerungen, welche spontan oder aber gezielt nacheinander ablaufen. Oft laufen dabei bevorzugt sigmatrope Umlagerungen unter gleichbleibenden Reaktionsbedingungen nacheinander ab und eignen sich daher besonders für die Durchführung entsprechend dem vorliegenden Verfahren in einem Dünnschichtverdampfer.

Wie anhand von Beispielen 2 und 7 (Citral) gezeigt werden konnte, eignet sich das hierin beschrieben Verfahren sowohl für die Durchführung von einzelnen [3,3]-sigmatropen Umlagerungen als auch von mehreren nacheinander ablaufender [3,3]-sigmatroper Umlagerungen.

Dabei besonders bevorzugt sind Tandem-Umlagerung, in der Reihenfolge Claisen-Cope.

Daher betrifft die vorliegende Erfindung in einer nächsten Weiterbildung des ersten Erfindungsgegenstandes ein Verfahren worin mindestens eine [3,3]-sigmatrope Umlagerung durchgeführt wird.

Es hat sich überraschend gezeigt, dass diese [3,3]-sigmatrope Umlagerungen einzeln oder in Kombination besonders selektiv nach dem vorliegenden Verfahren ablaufen. Die daraus resultierenden Produkte können in hohen Ausbeuten und mit hoher Reinheit isoliert werden.

In einer weiter bevorzugten Ausgestaltung der vorliegenden Erfindung, betrifft das erfindungsgemäße Verfahren zur Durchführung von thermischen Umlagerungsreaktionen daher die Durchführung von gekoppelten Claisen- und Cope-Umlagerungen in allen möglichen Kombinationen.

Somit beschreibt die vorliegende Erfindung in einer bevorzugten Ausführungsform eine technologische Optimierung der erfindungsgemäßen Claisen-Umlagerung basierend auf einem verbesserten Destillationsverfahren unter reduzierter thermischer Belastung. Formal ermöglicht diese apparative und somit verfahrenstechnische Verbesserung die lösungsmittelfreie Durchführung in zwei chemischen Reaktionsschritten, einerseits die thermische Umlagerung und andererseits die destillative Aufreinigung, unter den Bedingungen einer Reaktivdestillation innerhalb kurzer Zeit und geringem Kostenaufwand.

Eine weitere bevorzugte Variante des ersten Aspekts beschreibt ein erfindungsgemäßes Verfahren, weiterhin umfassend in Schritt a), die Bereitstellung von mindestens einer Säure als Reagenz.

Besonders bevorzugt eingesetzte Säuren sind ausgewählt aus der Gruppe, die besteht aus: organischen Säuren und vorzugsweise aus aliphatischen Carbonsäuren, substituierten Carbonsäuren, heterocyclischen Carbonsäuren sowie aromatischen Carbonsäuren und deren sauren Salzen, wobei insbesondere aromatische Carbonsäuren zu bevorzugen sind und insbesondere dabei die Salicylsäure.

Viele Umlagerungsreaktionen können durch die Verwendung von derartigen Katalysatoren beschleunigt werden oder aber selektiver durchgeführt werden.

Die Umsetzung der Reaktionsedukte zu den Reaktionsprodukten kann grundsätzlich aber auch ohne Katalysator, also nur durch Erhitzen, durchgeführt werden. Besonders bevorzugt und von Vorteil ist allerdings die Anwesenheit eines sauren Katalysators wie hierin beschrieben. Als saure Katalysatoren sind daher hierbei insbesondere die zuvor genannten Säuren und deren saure Salze geeignet, wobei vorzugsweise organische Säuren und deren Salze Verwendung finden.

Vorzugsweise wird die Säure als Reagenz zu den Reaktionsedukten hinzugegeben und anschließend die homogene Reaktionsmischung aus einem Vorratsgefäß über eine Dosierpumpe am oberen Teil der Rührblätter des Dünnschichtverdampfers eingespeist.

Thermische Umlagerungsreaktionen, wie beispielsweise Umlagerungen des Claisen-Typs, finden oft nur unter dem Einsatz von sehr hohen Temperaturen statt. Durch Verwendung von saurer Katalyse lassen sich diese Reaktionen unter entsprechend milderen Temperaturen durchführen, meist werden hierzu allerdings starke Säuren, wie Phosphorsäure, oder Lewis-Säuren eingesetzt.

Die hierin beschriebenen bevorzugten Säuren zeichnen sich durch ihre gute Löslichkeit in organischen Systeme und ihre geringere Tendenz zur Korrosionsbildung aus und eignen sich daher insbesondere für den Einsatz in dem vorliegenden, schonenderen Verfahren innerhalb eines Dünnschichtverdampfers.

Es hat sich gezeigt, dass die ohnehin milden Verfahrensbedingungen weiter abgemildert werden konnten, was zu einer noch schonenderen Behandlung der Reaktionsteilnehmer und insbesondere der Reaktionsprodukte führt. So können die hierin beschriebenen Umlagerungen zu den gewünschten Reaktionsprodukten kosteneffizient, ohne großen Energieaufwand, und in hohen Selektivitäten durchgeführt werden.

Insbesondere bevorzugt ist dabei die Bereitstellung von Salicylsäure als Reagenz in Schritt a) des erfindungsgemäßen Verfahrens zur Durchführung von thermischen Umlagerungsreaktionen in einem Dünnschichtverdampfer unter destillativen Bedingungen.

Es konnte ferner beobachtet werden, dass der Einsatz von Säuren, ausgewählt aus der Liste, die besteht aus: organischen Säuren und vorzugsweise aus aliphatischen Carbonsäuren, substituierten Carbonsäuren, heterocyclischen Carbonsäuren sowie aromatischen Carbonsäuren und deren sauren Salzen, die Selektivität der Umlagerungen, und dabei insbesondere von Claisen-Umlagerungen, erhöht, sodass kaum Nebenprodukte bei der Durchführung der Umlagerungen anfallen. Somit konnten signifikant verbesserte chemische Bedingungen für die Durchführung von Claisen-Umlagerungen festgestellt und eine Selektivitätssteigerung der Umlagerung von circa 70 % auf über 96 % beobachtet werden.

Eine derartige Steigerung der Selektivität trägt einen weiteren entscheidenden Beitrag zur Steigerung der Wirtschaftlichkeit des Herstellungsverfahrens bei.

Besonders selektive und daher effiziente Umsetzungen ließen sich somit in Zusammenhang mit Umlagerungen nach Claisen beobachten. Die besonders schonenden Reaktionsbedingungen als auch die Verwendung von Säuren wie hierin beschrieben führen zu sehr reinen Produkten in hohen Ausbeuten.

Eine bevorzugte Ausgestaltung der vorliegenden Erfindung betrifft die Durchführung von thermischen Umlagerungsreaktionen, umfassend mindestens eine Umlagerungen des Claisen-Typs, wobei die Reaktion unter dem Einsatz von Salicylsäure durchgeführt wird.

Ein Verfahren nach dem ersten Aspekt und den vorangehenden Weiterbildungen oder Varianten, welches weiterhin in Schritt a), die Bereitstellung von Salicylsäure als Reagenz für thermische Umlagerungen des Claisen-Typs umfasst, stellt somit eine weitere bevorzugte Weiterbildung der vorliegenden Erfindung dar.

Die Menge der eingesetzten Säure ist jeweils nachdem herzustellenden Produkt zu bestimmen. Vorzugsweise werden jedoch lediglich katalytische Mengen eingesetzt. Diese Mengen variieren beispielsweise von 1 mol-% bis 10-% und betragen beispielsweise vorzugsweise circa 5 mol-%.

In einer nächsten Variante des zuvor beschriebenen Verfahrens, ist das Produkt aus Schritt c) die Verbindung 3-(4-Isopropylcyclohexen-1-yl)propanal der Formel (I): 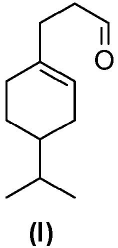 sowie deren Stereoisomere oder Gemische daraus. Insbesondere bevorzugt sind ferner die Stereoisomere, insbesondere Enantiomere und Diastereomere, der Verbindung der Formel (I) einzeln oder in Mischungen.

Wie nachfolgend näher erläutert wird, lässt sich die Verbindung der Formel (I) durch ein Verfahren umfassend mindestens die Schritte a), b) und c) wie hierin beschrieben, synthetisieren. Hierzu wird von den entsprechenden Acetalen als Reaktionsedukte ausgegangen, wobei diese Acetale über eine sauer katalysierte Addition eines geeigneten Alyllalkohols an einen Allylvinylether bereitgestellt werden.

Synthesen der Verbindung 3-(4-Isopropylcyclohexen-1-yl)propanal der Formel (I) sind bereits in EP 2578671 A1 beschrieben und umfassen die folgenden Schritte:
i) Durch Reaktion eines entsprechenden Allylalkohols mit einem entsprechenden Alkylvinylether in Gegenwart einer protischen Säure wie z. B. Phosphorsäure wird das entsprechende Acetal der Formel (II) hergestellt.
ii) Durch Umsetzung des Acetals mit katalytischen Mengen an Säure wie z. B. Hexansäure in einem hochsiedenden Lösungsmittel wird über ein Vinylether als Zwischenprodukt das gewünschte Aldehyd der Formel (I) erhalten.

Problematisch bei der beschriebenen Claisen-Umlagerung ist allerdings die geringe Selektivität, da bei der sauren Spaltung des Acetals (II) ebenfalls 5-Isopropyl-2-methylen-cyclohexanol (III) gebildet wird.

Dies wird in dem nachfolgenden Reaktionsschema verdeutlicht:

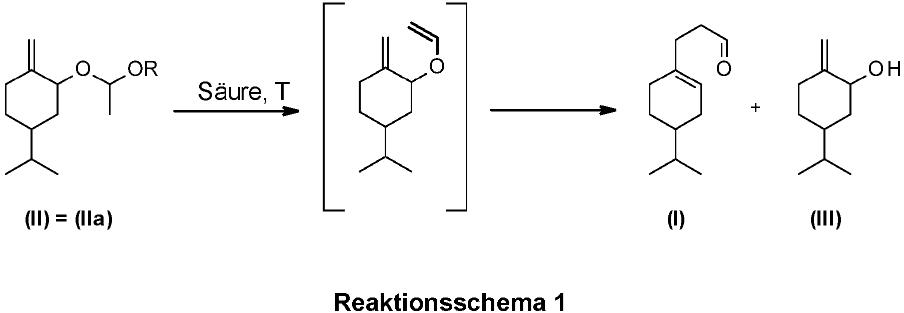

Der Einsatz von Salicylsäure als Katalysator für die vorliegende Claisen-Umlagerung führt zu einer deutlich höheren Selektivität der Reaktion, sodass bei der sauren Spaltung des Acetals (II) die Bildung von 5-Isopropyl-2-methylen-cyclohexanol (III) weitestgehend unterdrückt wird, und das Produkt in einer hohen Ausbeute mit hoher Reinheit erhalten wird, sodass die Wirtschaftlichkeit der Herstellung gesteigert werden kann.

Es ist bekannt, dass Riechstoffe der Verbindung 3-(4-Isopropylcyclohexen-1-yl)propanal der Formel (I) thermisch labile Eigenschaften aufweisen. Kurze Verweilzeiten und damit verbundene reduzierte thermische Belastungen, wie hierin beschrieben, ermöglichen es thermischen Zersetzungsprozessen entgegenzuwirken und eine hohe Produktqualität zu gewährleisten. So werden unerwünschte thermische Zersetzungsprodukte vermieden, welche nachteilig mit den Produkten wechselwirken können oder unangenehme Nebengerüche hervorrufen können oder den charakteristischen Geruchseindruck verfälschen oder nachteilig beeinflussen können oder sich nachteilig auf die Stabilität der Riechstoffe auswirken.

Das erfindungsgemäße Verfahren ermöglicht somit Produkte möglichst rein und frei von Verunreinigungen, d. h. möglichst selektiv zu synthetisieren und eigenen sich daher insbesondere für die Herstellung von qualitativ hochwertigen und stabilen Riech- oder Duftstoffen.

Beispiel 8 der Offenbarung EP 2578671 A1 betreffend die Synthese von Synthese von 3-[(4R)-4-Isopropylcyclohexen-1-yl]propanal zeigt, dass das darin beschriebene Verfahren das gewünschte Produkt lediglich in einer Reinheit von 52 % bereitstellt. Dies entspricht 58 % der theoretisch möglichen Ausbeute. Erste eine weitere Aufreinigung liefert eine Reinheit von 96,5 %. Daher ist schließlich nach der zweiten Aufreinigung mit einer deutlich geringeren Ausbeute von circa 30% zu rechnen. Weiterhin ist anzumerken, dass die darin verwendete Hexansäure als giftig eingestuft wird.

Wie Beispiel 2 der vorliegenden Erfindung entnommen werden kann, wird bereits direkt nach der Synthese ein deutlich reineres Produkt mit einer Reinheit von 72 % erhalten. Eine anschließende Aufreinigung ergibt schließlich ein noch reineres Produkt mit 98,2 %-iger.

Somit kann also beobachtet werde, dass ein Verfahren entsprechend der vorliegenden Erfindung gegenüber gängigen Herstellungsverfahren deutlich reinere Produkte in einem größeren Maßstab liefert. Außerdem wird die Verwendung giftiger Chemikalien durch deutlich ungefährlichere Substanzen ersetzt.

Ein direkter Vergleich destillativer Verfahren zeigt, dass das erfindungsgemäße Verfahren unter deutlich milderen Bedingungen operiert (Vergleich Beispiel 2 und 3) und ferner, dass der Einsatz von deutlich umweltgefährlichen, luft- und lichtempfindlichen Substanzen, wie Trioctylamin und Dibenzylether, vermieden werden kann.

Das vorliegend beschriebene Verfahren läuft dabei gemäß den nachfolgendem Reaktionsschema (Weg A und/oder Weg B) ab:

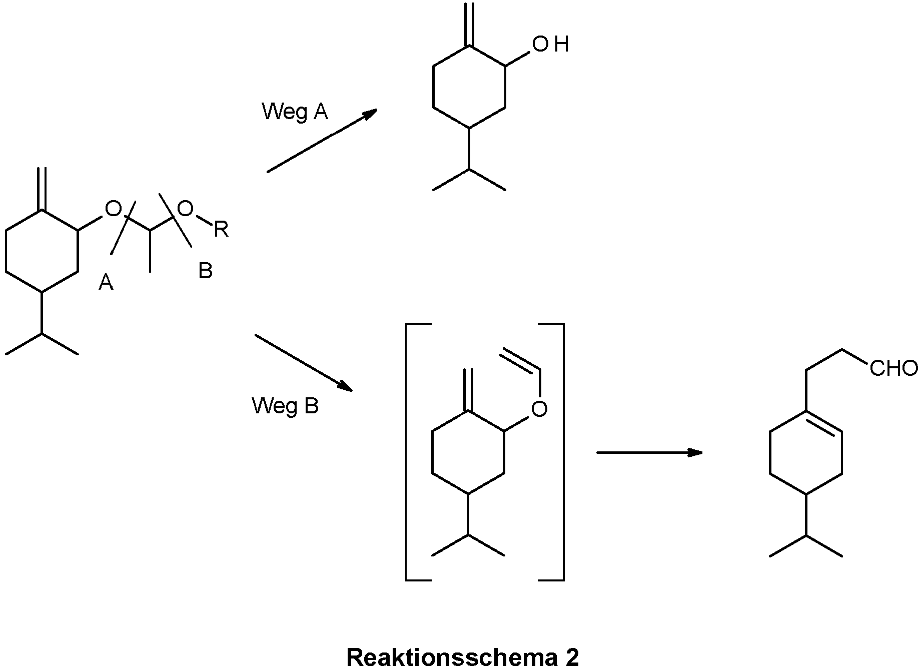

Das aus der Reaktion gemäß "Weg A" erhaltene Produkt entspricht einer Rückgewinnung des Edukts, welches erneut umgesetzt werden kann. Basierend auf dem vorliegenden Verfahren können somit potentiell anfallende Nebenprodukte effizient wiedereingesetzt werden und somit hohe Ausbeuten, hohe Reinheiten, hohe Selektivitäten und geringe Mengen an unerwünschten Nebenprodukten erzielt werden.

Ausgehend von der schonenden Umsetzung aufgrund einer geringen thermischen Belastung lässt sich die Selektivität und Reinheit maximieren ohne Einbußen in der Ausbeute zu bedingen.

Ferner kann festgestellt werden, dass die milden Reaktionsbedingungen des erfindungsgemäßen Verfahrens aufgrund des geringen Energieaufwandes zu bevorzugen sind und in steuerbaren und einheitlichen Selektivitäten resultieren. Kurze Verweilzeiten sowie der Einsatz von Säurekatalysatoren ermöglichen eine Steigerung der Raum-Zeit Ausbeute und reduzieren die thermische Belastung der chemischen Erzeugnisse, welche sich oft, beispielsweise aufgrund von thermischen Zersetzungen, negativ auf die Qualität der Erzeugnisse auswirkt.

Außerdem kann festgestellt werden, dass im Vergleich zu Beispiel 3 deutlich weniger Reaktionsteilnehmer am Verfahren beteiligt sind, der Bedarf an Chemikalien geringer ist und somit eine deutliche Ersparnis im Hinblick auf Zeit, Kosten und Ressourcen mit den erfindungsgemäßen Verfahren erzielt werden kann.

Daher konnte gezeigt werden, dass das vorliegende Verfahren deutlich materialeffizienter gegenüber gängigen Herstellungsverfahren ist sowie gegenüber anderen destillativen Verfahren wie der Reaktivdestillation, wie in den hierin beschriebenen Beispielen dargestellt.

In einer weiteren Weiterbildung des erfindungsgemäßen Verfahrens sind die Reaktionsedukte ausgewählt aus der Gruppe, umfassend die Acetale der Formeln (**IIa**), (**IIb**) und/oder (**IIc**): 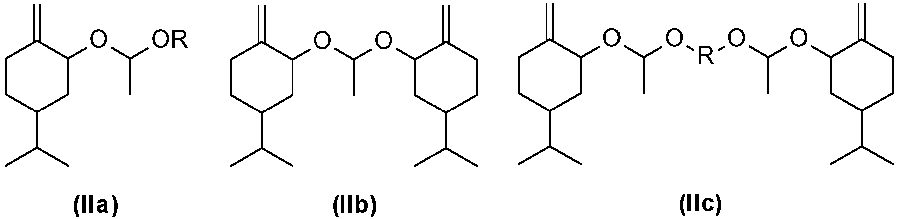 worin der Rest R in den Verbindungen der Formel (**IIa**) für lineare oder verzweigte Alkylgruppen C₁₋₂₀, Benzylgruppen, Acetylgruppen, Phenylgruppen, -CH₂-CH₂-OCH₃, -CH(CH₃)-CH₂-OCH₃, -(CH₂)₂-O-(CH₂)₂-O-CH=CH₂, -(CH₂)₂-O-(CH₂)₂-O-(CH₂)₂-O-CH=CH₂ oder 2-Methylen-tetrahydrofuran steht, und in Verbindungen der Formel (**IIc**) für lineare oder verzweigte Alkylgruppen C₁₋₂₀, -(CH₂)₂-O-(CH₂)₂-, oder -(CH₂)₂-O-(CH₂)₂-O-(CH₂)₂- steht, sowie deren Stereoisomere, insbesondere Diastereomere und Enantiomere, sowie Mischungen daraus.

Die Verbindungen der Formel **(IIa),** Formel (**IIb**) oder der Formel (**IIc**) können in verschiedenen Formen entsprechend der möglichen Konstitutionsisomere (Regioisomere) für die Reste R, sowie Stereoisomere, insbesondere Enantiomere, Diastereomere, der Formel **(IIa),** Formel (**IIb**) oder der Formel (**IIc**) vorliegen, sowie als Mischungen der Stereoisomere in jedem beliebigen Mischungsverhältnis.

Überraschenderweise hat sich gezeigt, dass der Einsatz des Dünnschichtverdampfers als Reaktor die Herstellung von 3-(4-Isopropylcyclohexen-1-yl)propanal (**I**), ausgehend von Acetalen der Gestalt (**IIa**), (**IIb**) oder **(IIc),** ermöglicht.

Durch Funktionalisierung der Seitenketten mit den hierin beschriebenen bevorzugten Resten konnte eine weitere Steigerung der Selektivität während der Umlagerung der Acetale zu den gewünschten Produkten beobachtete werden.

Besonders starke Selektivitätssteigerungen konnten in Zusammenhang mit der hierin beschriebenen Säurekatalyse während der Claisen-Umlagerung beobachtete werden.

Besonders hohe Selektivitätssteigerungen und damit verbunden höhere Reinheit und Ausbeute, konnten dabei in Bezug auf Ethylgruppen, Butylgruppen und -(CH₂)₂-O-(CH₂)₂-O-CH=CH₂ als Reste R beobachtet werden.

Eine alternative Weiterbildung des erfindungsgemäßen Verfahrens betrifft daher die Reaktionsedukte der Formel **(IIa),** worin die Reste R der Acetale der Formel (Ila) vorzugsweise Ethylgruppen, Butylgruppen und -(CH₂)₂-O-(CH₂)₂-O-CH=CH₂ sind.

In einer bevorzugten Ausgestaltung ist hierin der Rest R der Acetale der Formel (**IIa**) insbesondere bevorzugt -(CH₂)₂-O-(CH₂)₂-O-CH=CH₂.

Die zuvor genannte Ausgestaltung zeigt den größten selektivitätssteigernden Effekt und ist daher besonders bevorzugt im Rahmen der vorliegenden Erfindung.

In einer nächsten Variante betrifft die vorliegende Erfindung ein Verfahren, weiterhin umfassend einen zusätzlichen/vorangehenden Verfahrensschritt 0) vor dem Schritt a), wobei in diesem Schritt 0) die Reaktionsedukte der Formeln (**IIa**), (**IIb**) und/oder **(IIc)** über sauer katalysierte Addition von 5-Isopropyl-2-methylen-cyclohexanol (Formel **(III))** an die entsprechenden Vinylether der Formel **(IV)** bereitgestellt werden: 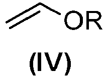 worin der Rest R für eine lineare oder verzweigte Alkylgruppe C₁₋₂₀, Benzylgruppen, Acetylgruppen, Phenylgruppen, -CH₂-CH₂-OCH₃, -CH(CH₃)-CH₂-OCH₃, (CH₂)₂-O-(CH₂)₂-O-CH=CH₂, (CH₂)₂-O-(CH₂)₂-O-(CH₂)₂-O-CH=CH₂ oder 2-Methylen-tetrahydrofuran steht.

Schritt 0) des erfindungsgemäßen Verfahrens betrifft daher eine Möglichkeit zur Herstellung der erfindungsgemäßen Reaktionsedukte, d. h. der entsprechenden Acetale der Formel **(II)** wie hierin beschrieben, durch saure Umsetzung der bekannten Verbindung 5-Isopropyl-2-methylen-cyclohexanol der Formel **(III)** mit Vinylethern der Formel **(IV).**

Die daraus erhaltenen Acetale **(II)** werden sauer katalysiert in die entsprechenden Vinylether gespalten, die direkt thermisch in das gewünschte Aldehyd 3-(4-Isopropylcyclohexen-1-yl)propanal der Formel **(I)** mittels Claisen-Umlagerung überführt werden.

Wie hierin beschrieben ist die Umsetzung von Acetalen der Formeln (**IIa**), (**IIb**) und **(IIc)** wie hierin beschrieben zu 3-(4-Isopropylcyclohexen-1-yl)propanal der Formel **(I)** gemäß dem erfindungsgemäßen Verfahren deutlich selektiver gegenüber Verfahren des Standes der Technik, wodurch deutlich höhere Ausbeuten und reinere Produkte gewonnen werden können. Nichtsdestotrotz kein ein gewisser kleiner Anteil an Nebenprodukten wie dem hierin beschriebenen 5-Isopropyl-2-methylen-cyclohexanol der Formel **(III)** gemäß Reaktionsschema 1 anfallen. Die Kombination des hierin beschriebenen Verfahrens aus thermischer Umsetzung und gleichzeitiger destillativer Aufreinigung der Reaktanden ermöglicht es, ein derartiges Nebenprodukt als Ergebnis des Destillationsprozesses in weitestgehend reiner Form aufzufangen und anschließend erneut in Schritt 0) für die Herstellung der genannten Reaktionsedukte der Formeln (**IIa**), (**IIb**) und **(IIc)** wiedereinzusetzen und gewährleistet somit einen materialeffizient Einsatz der verwendeten Rohstoffe.

Dies unterstreicht somit den umwelt-, ressourcen- und kostenschonenden Charakter des erfindungsgemäßen Verfahrens.

Gemäß einer weiteren Weiterbildung des erfindungsgemäßen Verfahrens werden als Säurekatalysatoren für die sauer katalysierte Addition in Schritt 0) vorzugsweise Lewis-Säuren oder Brønstedt-Säuren eingesetzt.

Als Säuren für die sauer katalysierte Addition werden dabei bevorzugt Lewis-Säuren und Brønstedt-Säuren eingesetzt. Insbesondere Magnesiumsalze, wie z. B. Magnesium(II)chlorid, Magnesium(II)sulfat, Ammoniumsulfat, Phosphorsäure, Zeolithe sind hierbei bevorzugt zu verwenden.

Die hierin verwendeten Säuren als Katalysatoren führen zu einer erhöhten Selektivität der Additionsreaktion.

In einer weiteren Variante der vorliegenden Erfindung und somit auch des erfindungsgemäßen Verfahrens, werden für die sauer katalysierte Addition von 5-Isopropyl-2-methylen-cyclohexanol (Formel (**III**)) an die entsprechenden Vinylether der Formel **(IV)** in Schritt 0) des erfindungsgemäßen Verfahrens, die entsprechenden Vinylether der Formel **(IV)** in einem 1,1 bis 5,0-fachem molaren Überschuss eingesetzt.

Der Einsatz der Vinylether der Formel **(IV)** in einem definierten molaren Überschuss beeinflusst dabei die Selektivität der Bildung der Acetale, d. h. der Reaktionsedukte der Formeln (**IIa**) und **(IIc).**

Insbesondere der Einsatz der Vinylether der Formel **(IV)** in einem 1,2 bis 2,0-facher molaren Überschuss ist zu bevorzugen.

Für den Einsatz der Vinylether der Formel **(IV)** in einem derartigen Überschuss sind dabei die größten selektivitätssteigernden Effekte der hierin beschriebenen Additionsreaktion zu den Reaktionsedukten zu beobachten.

In diesem Zusammenhang besonders bevorzugt ist es den, im Reaktionsgemisch zurückbleibenden Vinylether der Formel (IV), destillativ zurückzugewinnen.

Nicht umgesetzter oder überschüssiger Vinylether der Formel (IV) aus Schritt 0) des vorliegenden Verfahrens wird vorzugsweise destillativ zurückgewonnen. Besonders bevorzugt ist dabei eine Destillation im Vakuum.

Vakuumdestillation erfordern geringere Temperaturen und finden daher als besonders schonendes Verfahren unter Anwendung von moderaten Temperaturen Verwendung.

Eine destillative Wiederaufbereitung des Vinylethers der Formel **(IV)** aus Schritt 0) ermöglicht den erneuten Einsatz des Vinylethers in anschließenden sauer katalysierten Umsetzungen mit 5-Isopropyl-2-methylen-cyclohexanol der Formel **(III)** für die Herstellung weiterer Reaktionsedukte der Formeln (**IIa**), (**IIb**) und **(IIc)** oder aber für andere Zwecke.

Dieser materialeffiziente Einsatz von Rohstoffen trägt entscheidend zur Wirtschaftlichkeit des hierin beschriebenen Verfahrens bei. Ein solcher Wiedereinsatz schont die vorhandenen Ressourcen und leistet einen entscheidenden Beitrag für einen nachhaltigen und umweltschonenden Verfahrensprozess während gleichzeitig anfallende Produktions- und Entsorgungskosten minimiert werden können.

In einer besonders bevorzugten Weiterbildung des hierin beschriebenen Verfahrens, läuft das Verfahren als kontinuierlicher Prozess ab.

Kontinuierliche Verfahren finden insbesondere bei der Verarbeitung großer Rohstoffmengen Anwendung und zeichnen sich durch einen kontinuierlichen, d. h. ohne Unterbrechungen ablaufenden Prozess aus. Kontinuierliche Verfahrensprozesse ermöglichen es, die Anzahl von Zwischenschritten, wie das Wiederbefüllen oder Abkühlen und Aufheizen der Apparatur, zu reduzieren und sind daher im Vergleich zu Chargenprozessen wirtschaftlicher.

Weiterhin gewährleisten kontinuierliche Herstellungsverfahren eine konstante, d. h. gleichbleibende Produktqualität, ohne dabei Qualitätsschwankungen zwischen einzelnen Chargen zu zeigen.

In kontinuierlichen Verfahren stellt sich dabei ein konstantes Fließgleichgewicht ein. Das Gleichgewicht der Reaktion wird durch konstantes Abtrennen der Produkte in Richtung höherer Ausbeuten verschoben. Ferner ermöglicht eine kontinuierliche Abtrennung von nichtumgesetzten Edukten oder Nebenprodukten durch den destillativen Charakter der vorliegenden Erfindung eine höhere Reinheit der Produkte zu erzielen, da somit die Wahrscheinlichkeit möglicher Nebenreaktionen deutlich verringert wird.

Insgesamt weisen kontinuierliche Verfahren einen deutlichen ökonomischen Vorteil gegenüber Chargenprozessen auf und eignen sich insbesondere für Herstellungsverfahren im industriellen Maßstab und sind daher im Rahmen der vorliegenden Erfindung besonders bevorzugt.

Um die thermischen Umlagerungsreaktionen in einem kontinuierlichen oder semikontinuierlichen Prozess durchzuführen, kann der hierin beschriebenen Dünnschichtverdampfer zu einem Kreislaufreaktor erweitert werden. Hierzu werden Reaktionsedukte wieder zurück in das Vorratsgefäß gepumpt und über die Dosierpumpe wieder am oberen Teil der Rührblätter des Dünnschichtverdampfers eingespeist. Dies führt zu einer Verlängerung der Reaktionszeit bei gleichzeitig verkürzter Verweilzeit, sodass eine vollständige Umsetzung der Reaktionsedukte gewährleistet werden kann.

Insbesondere ist es bevorzugt, die abdestillierten Stoffe erneut einem destillativen Trennungsprozess zu unterziehen bevor diese wieder dem System zugeführt werden. Hierzu bietet es sich an, Apparaturen geeignet zur fraktionierten Destillation, zwischenzuschalten und nur die entsprechenden Reaktionsedukte wieder in den Dünnschichtverdampfer zurückzuleiten.

So können nichtumgesetzten Reaktionsedukte wieder dem Herstellungsprozess zurückgeführt werden, um die anfallende Menge an umweltbelastenden Reaktionsnebenprodukten zu reduzieren und gleichzeitig einen material- und kosteneffizienten Einsatz der Rohstoffe zu gewährleisten.

Ferner ist zu betonen, dass kontinuierliche Verfahrensprozessen der Regel aufgrund der konstanten Reaktionsbedingungen stabiler und gleichmäßiger ablaufen und dadurch höhere Selektivitäten der Reaktionen gewährleisten, wodurch die Reinheit und Ausbeute der Produkte gesteigert werden kann.

Ferner beschreibt eine bevorzugte Weiterbildung der vorliegenden Erfindung ein Verfahren, umfassend die Rückgewinnung von 5-Isopropyl-2-methylen-cyclohexanol der Formel (**III**), anderen Ausgangsverbindungen und/oder nicht umgelagerten Reaktionsedukten des Verfahrens.

Im Zuge der erfindungsgemäßen Reaktivdestillation, in der das Produkt im Auffangkolben angereichert wird, können nicht umgesetzte Reaktionsedukte oder Vorstufen und Nebenprodukte destillativ entfernt werden und anschließend dem Verfahren wieder zugeführt werden.

Insbesondere ist dabei die Rückgewinnung der Verbindung 5-Isopropyl-2-methylen-cyclohexanol der Formel (III) bevorzugt. Diese Verbindung, welche trotz der hierin beschriebenen Selektivitätssteigerung der Umsetzung, als mögliches Nebenprodukt anfallen kann, kann anschließend erneut für die Herstellung der Reaktionsedukte in Schritt 0) des Verfahrens wiedereingesetzt werden.

Dies schont die Ressourcen und spart gleichzeitig Kosten für Entsorgung und Wiederbeschaffung derselben Verbindung.

Die hierin beschriebene bevorzugte Rückgewinnung von Rohstoffen ermöglicht einen optimierten und effizienten Materialeinsatz in der Produktion unter Berücksichtigung von Umweltgesichtspunkten und ermöglicht eine Reduzierung der anfallenden Herstellungskosten.

In einem zweiten Aspekt betrifft die vorliegende Erfindung schließlich das Produkt unmittelbar hergestellt aus dem erfindungsgemäßen Verfahren wie hierin beschrieben.

Wie vorangehend erläutert liefert das hierin beschriebene Verfahren Produkte mit ausgezeichneter Reinheit in hoher Ausbeute basierend auf einem optimierten und hoch selektiven Herstellungsprozess unter destillativen Bedingungen. Gelichzeitig können die hierin beschrieben Produkte in großen Maßstab kostengünstig und umweltschonend produziert werden.

Die hohe Wirtschaftlichkeit des Verfahrens und der daraus resultierenden Produkte ermöglichen es die hohe Nachfrage kostengünstig und vollständig zu decken.

In einer bevorzugten Ausgestaltung ist das Produkt unmittelbar hergestellt aus dem erfindungsgemäßen Verfahren wie hierin vorzugsweise die Verbindung 3-(4-Isopropylcyclohexen-1-yl)propanal der Formel (I), welche insbesondere Anwendung als Riechstoff mit Maiglöckchenduft findet.

### Beispiele

Die vorliegende Erfindung wird nachstehend anhand von Ausführungsbeispielen näher beschrieben. Zunächst beziehen sich die angeführten Beispiele auf die Herstellung von Verbindungen der Formel (I) und besonders bevorzugter Verbindungen. Weiterhin ist zu beachten, dass die IUPAC Nomenklatur von der bisher verwendeten generischen Bezeichnung abweichen kann.

Für Spektroskopische Daten gilt im Folgenden die englischsprachige Regelung hinsichtlich der Verwendung von Punkten als Trennzeichen bei Zahlenangaben um eine bessere Übersichtlichkeit der Messergebnisse zu gewährleisten. In diesem Zusammenhang sind in Angaben der Form "δ = 7.12 (dd, J = 3.7, 0.9 Hz, 2H)" die Messwerte zu lesen als "δ = 7,12", "3,7" und "0,9". Dabei wird für die NMR-Daten die alternative Punkt-Komma Regel angewandt.

In den Beispielen 2 und 3 wird im Folgenden die Synthese der Verbindung 3-(4-Isopropylcyclohexen-1-yl)propanal der Formel (I) ausgehend vom selben Edukt 4-Isopropyl-1-methylen-2-[1-[2-(2-vinyloxyethoxy)ethoxy]ethoxy]cyclo-hexan aus Beispiel 1 beschrieben.

### Beispiel 1: Acetal

### Synthese von 4-Isopropyl-1-methylen-2-[1-[2-(2-vinyloxyethoxy)ethoxy]ethoxy]-cyclohexan (Reaktionsedukt)

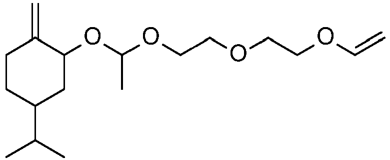

In einem 2 L-Dreihalsreaktionskolben mit Tropftrichter, Intensivkühler und Magnetrührer werden 500,00 g (2,91 mol, 89,8 % Reinheit) 5-Isopropyl-2-methylene-cyclohexanol und 1,68 g (0,01 mol) 85 %-ige Phosphorsäure vorgelegt und auf 30 °C erwärmt. Daraufhin werden 923,18 g (5,82 mol) Diethyleneglycoldivinylether so zugetropft, sodass die Temperatur nicht über 40 °C steigt. Die Reaktion wird bei gleicher Temperatur weitere 4 Stunden bis zum vollständigen Umsatz gerührt und anschließend auf Raumtemperatur gekühlt. Anschließend wird die Reaktionsmischung in 1,3 L *tert*-Butyl-methylether aufgenommen und mit 1,3 L einer gesättigten Natriumcarbonat-Lösung 20 min lang gerührt. Die Phasen werden getrennt und die organische Phase wird mit 1,0 L einer gesättigten Natriumcarbonat-Lösung gewaschen. Die wässrigen Phasen werden einmalig mit 800 mL *tert*-Butyl-methylether extrahiert. Die organischen Phasen werden vereinigt und über Natriumsulfat getrocknet und anschließend filtriert. Die flüchtigen Bestandteile, das Lösungsmittel und überschüssiger Diethylenglycoldivinylether (Sdp. 60 °C bei p = 2,5 mbar) werden schließlich im Vakuum destilliert. Die auf diesem Wege erhaltenen 914,2 g Rückstand werden als das Rohprodukt 4-Isopropyl-1-methylen-2-[1-[2-(2-vinyloxyethoxy)ethoxy]ethoxy]cyclohexan direkt in der Folgereaktion (Beispiele 2 und 3) eingesetzt.

### Spektroskopische Daten:

EI-MS m/z (%): 268 (1, [M-44]⁺), 225 (2), 180 (4), 162 (19), 137 (65), 115 (58), 110 (54), 93 (50), 87 (100), 81 (85), 68 (85), 55 (19), 43 (77), 29 (15).

¹H-NMR (400 MHz, CDCl₃, 300 K): δ = 7.18 - 7.15 (m, 1H), 7.13 (dd, J = 4.8, 1.9 Hz, 1H), 7.07 (d, J = 1.7 Hz, 1H), 7.07 - 7.03 (m, 1H), 4.88 (s, 2H), 1.68 (s, 6H) ppm.

¹³C-NMR (101 MHz, CDCl₃, 300 K): δ = 206.99, 192.78, 191.80, 191.67, 163.50, 133.88, 132.25, 132.13, 131.98, 130.04, 129.15, 128.86, 128.60, 128.56, 127.95, 127.26, 126.32, 125.51, 124.50, 123.52, 86.16, 82.02, 70.23, 64.25, 63.17, 63.15, 30.92, 29.47, 29.12 ppm.

### Beispiel 2: DV-Reaktion

### Synthese von 3-(4-Isopropylcyclohexen-1-yl)propanal der Formel (I) unter Verwendung des Dünnschichtverdampfers nach dem erfindungsgemäßen Verfahren

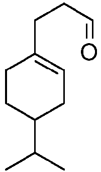

Die Reaktionsmischung wird aus einem Vorratsgefäß über eine Dosierpumpe am oberen Teil der Rührblätter des Dünnschichtverdampfers eingespeist. Der Dünnschichtverdampfer wird mit Kühlung und Kopfabnahme, regulierbarer Einstellung der Rotorblätter, einer Vakuumpumpe zur Einstellung des Drucks und einem Auffangglaskolben am unteren Ende ausgestattet.

Im Auffangkolben werden zur Pufferung des pH-Wertes 170,00 g einer 5 %-igen wässrigen Natriumcarbonat-Lösung vorgelegt. Als Rotationsgeschwindigkeit werden 500 U/min Rührgeschwindigkeit eingestellt und ein Vakuum von 800 mbar angelegt. Der Dünnschichtverdampfer wird auf T = 230 °C erwärmt. Im Vorratsgefäß, das über die Dosierpumpe entleert wird, werden 160,00 g 4-Isopropyl-1-methylen-2-[1-[2-(2-vinyloxyethoxy)ethoxy]ethoxy]cyclohexan aus Beispiel 1 und 8,00 g (57,34 mmol, 5,0 m%) Salicylsäure eingewogen und gerührt. Die Lösung wird mit einer Geschwindigkeit von 2,5 mL/min auf den Dünnschichtverdampfer aufgetragen. Während der Reaktionsdurchführung werden am Kopf der Kolonne Leichtsieder abdestilliert. Das Produkt 3-(4-Isopropylcyclohexen-1-yl)propanal der Formel **(I)** wird im Sumpf innerhalb des Auffangkolbens angereichert. Nach Beendigung der Reaktion wird das Gerät abgekühlt und entspannt. Die Reaktionslösung im Auffangkolben wird mit 150 mL tert-Butyl-methylether versetzt und extrahiert. Nach Phasenseparation wird erneut die wässrige Phase mit 150 mL tert-Butyl-methylether extrahiert und die Phasen anschließend separiert. Die vereinigten organischen Phasen werden mit 250 mL einer gesättigten Kochsalz-Lösung gewaschen. Nach Phasentrennung wird die organische Phase über Natriumsulfat getrocknet und anschließend filtriert. Das Lösungsmittel wird daraufhin im Vakuum entfernt. Es werden auf diesem Wege 119,04 g Rohprodukt mit einer Reinheit von 72 % erhalten.

Es werden 119,4 g (72 %-ige Reinheit) der Rohware 3-(4-Isopropylcyclohexen-1-yl)propanal der Formel **(I)** einer destillativen Aufreinigung an einer Spaltrohrkolonne unterzogen. Es werden dabei 77,4 g 3-(4-Isopropylcyclohexen-1-yl)propanal der Formel **(I)** mit einer Reinheit von 98,2 % isoliert (74 - 75 °C bei 1,0 mbar; R (Rücklauf) / D(Abnahme) = 100/1).

Die analytischen Daten entsprechen denen der Literatur **(**EP 2578671 A1**).**

### Beispiel 3: Reaktivdestillation

### Synthese von 3-(4-Isopropylcyclohexen-1-yl)propanal der Formel (I) unter den Bedingungen einer Reaktivdestillation

In einem 0,5L-Dreihalsreaktionskolben mit Liebig-Kühler, kleiner Kolonne und Magnetrührer werden 160g (= 0,509 mol Einsatz) 4-Isopropyl-1-methylen-2-[1-[2-(2-vinyloxyethoxy)-ethoxy]ethoxy]cyclohexan aus Beispiel 1 eingewogen und mit 0,67 g Lithium-dihydrogenphosphat, 0,67 g Trioctylamin und 160 g Dibenzylether versetzt. Anschließend wird die Reaktionsmischung bei 100 mbar auf 175 °C erhitzt und die Leichtsieder am Kopf der Kolonne 5 Stunden lang destilliert. Nach vollständigem Umsatz wird anschließend 3-(4-Isopropylcyclohexen-1-yl)propanal der Formel **(I)** (bei 175 °C, 1 mbar) vom Sumpf abdestilliert. Es werden dabei 275,1 g des Rohproduktes mit einer Reinheit von 24,3 % erhalten.

Es werden anschließend 275,1 g (24,3 %-ig) der Rohware 3-(4-Isopropylcyclohexen-1-yl)propanal der Formel **(I)** einer destillativen Aufreinigung an einer Spaltrohrkolonne unterzogen. Es werden dabei 61,5 g 3-(4-Isopropylcyclohexen-1-yl)propanal der Formel **(I)** mit einer Reinheit von 97,8 % isoliert (74 - 75 °C bei 1,0 mbar; R/D = 100/1).

Die analytischen Daten entsprechen denen der Literatur **(**EP 2578671 A1**).**

### Beispiel 4:

### Synthese von 4-Isopropyl-2-[1-(2-methoxy-1-methyl-ethoxy)ethoxy]-1-methylen-cyclohexan (Reaktionsedukt)

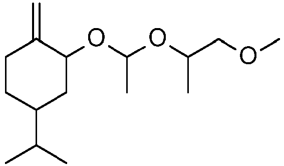

In einem 250 mL-Dreihalsreaktionskolben mit Tropftrichter, Intensivkühler und Magnetrührer werden 44,66 g (260 mmol, 89,8 % Reinheit) 5-Isopropyl-2-methylene-cyclohexanol und 0,15 g (1,3 mmol) 85 %-ige Phosphorsäure vorgelegt und auf 30 °C erwärmt. Daraufhin werden 61,14 g (520 mol) 1-Methoxy-2-vinyloxypropan so zugetropft, sodass die Temperatur nicht über 40 °C steigt. Die Reaktion wird bei gleicher Temperatur weitere 2,5 Stunden bis zum vollständigen Umsatz gerührt und anschließend auf Raumtemperatur gekühlt. Anschließend wird die Reaktionsmischung in 100 mL tert-Butyl-methylether aufgenommen und mit 100 mL einer gesättigten Natriumcarbonat-Lösung 10 min lang gerührt. Die Phasen werden getrennt und die organische Phase wird mit 100 mL einer gesättigten Natriumcarbonat-Lösung gewaschen. Die wässrigen Phasen werden einmal mit 100 mL tert-Butyl-methylether extrahiert. Die organischen Phasen werden vereinigt und über Natriumsulfat getrocknet und anschließend filtriert. Die flüchtigen Bestandteile, das Lösungsmittel und überschüssiges 1-Methoxy-2-vinyloxypropan werden schließlich im Vakuum über eine Kugelrohrapparatur destilliert (Sdp. 76 - 80 °C bei p = 0,6 mbar). Die auf diesem Wege erhaltenen 74,57 g Rückstand werden als das Rohprodukt 4-Isopropyl-2-[1-(2-methoxy-1-methyl-ethoxy)ethoxy]-1-methylen-cyclohexan (Reinheit 88 %) direkt in der Folgereaktion (Beispiel 5) eingesetzt.

Die analytischen Daten entsprechen denen der Literatur **(**EP 2578671 A1**).**

### Beispiel 5:

### Synthese von 3-(4-Isopropylcyclohexen-1-yl)propanal der Formel (I) unter den Bedingungen einer Reaktivdestillation

In einem 250 mL-Dreihalsreaktionskolben mit Liebig-Kühler, 8 cm lange Vigreux-Kolonne und Magnetrührer werden 74,00 g 4-Isopropyl-2-[1-(2-methoxy-1-methyl-ethoxy)ethoxy]-1-methylen-cyclohexan (88 %-ig) aus Beispiel 4 eingewogen und mit 311 mg Lithiumdihydrogenphosphat, 311 mg Trioctylamin und 74 g (71,15 mL) Dibenzylether versetzt. Anschließend wird die Reaktionsmischung bei 430 mbar auf 175 °C erhitzt und die Leichtsieder am Kopf der Kolonne 3 Stunden destilliert. Nach vollständigem Umsatz wird anschließend 3-(4-Isopropylcyclohexen-1-yl)propanal der Formel **(I)** (124 °C, 2,5 mbar) vom Sumpf abdestilliert. Es werden 100,22 g 3-(4-Isopropylcyclohexen-1-yl)propanal der Formel **(I)** in einer 27 %-igen Reinheit erhalten.

Die analytischen Daten entsprechen denen der Literatur **(**EP 2578671 A1**).**

### Beispiel 6:

### Synthese von 3-Methyl-1,1-bis(3-methylbut-2-enoxy)but-2-en (Reaktionsedukt)

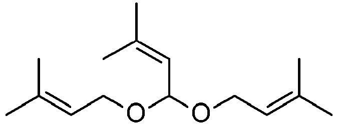

In einem 4 L-Dreihalsreaktionskolben mit Intensivkühler, Magnetrührer und Wasserabscheider werden 1226 g (13,95 mol) 3-Methylbut-2-en-1-ol, 4,02 g (35 mmol) 85 %-ige Phosphorsäure und 400 g (4,65 mol) 3-Methylbut-2-enal in 1,6 L Cyclohexan gelöst. Danach wird die Reaktionsmischung 30 Stunden lang unter Rückfluss erhitzt, das dabei entstehende Wasser abgetrennt und anschließend das Reaktionsgemisch auf Raumtemperatur abgekühlt. Anschließend wird die Reaktionsmischung in 500 mL tert-Butyl-methylether aufgenommen und in 800 mL einer 10 %-igen Natriumcarbonat-Lösung 10 min lang gerührt. Die Phasen werden getrennt und die organische Phase wird mit 800 mL einer 5 %-igen Natriumcarbonat-Lösung gewaschen. Die wässrigen Phasen werden einmal mit 500 mL *tert*-Butyl-methylether extrahiert. Die organischen Phasen werden vereinigt und über Natriumsulfat getrocknet und anschließend filtriert. Die flüchtigen Bestandteile und das Lösungsmittel werden im Vakuum entfernt. Das Rohprodukt wird über eine 12 cm Vigreux-Kolonne (Sdp. 72 - 107 °C bei p = 1,2 - 2,2 mbar) fraktionierend destilliert. Die auf diesem Wege erhaltenen 537 g von 3-Methyl-1,1-bis(3-methylbut-2-enoxy)but-2-en (Reinheit 79 %, Ausbeute 53 %) werden direkt in der Folgereaktion (Beispiel 7) eingesetzt.

Die analytischen Daten entsprechen denen der Literatur.

### Beispiel 7:

### Synthese von 3,7-Dimethylocta-2,6-dienal unter Verwendung des Dünnschichtverdampfers nach dem erfindungsgemäßen Verfahren

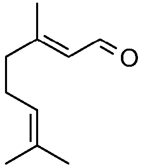

Eine Reaktionsmischung wird aus einem Vorratsgefäß über eine Dosierpumpe am oberen Teil der Rührblätter des Dünnschichtverdampfers eingespeist. Der Dünnschichtverdampfer wird mit Kühlung und Kopfabnahme, regulierbarer Einstellung der Rotorblätter, einer Vakuumpumpe zur Einstellung des Drucks und einem Auffangglaskolben am unteren Ende ausgestattet.

Als Rotationsgeschwindigkeit werden 500 U/min Rührgeschwindigkeit eingestellt. Der Dünnschichtverdampfer wird auf T = 230 °C erwärmt. Im Vorratsgefäß, das über die Dosierpumpe entleert wird, werden 100,00 g 3-Methyl-1,1-bis(3-methylbut-2-enoxy)but-2-en (79 % Reinheit, 331,7 mmol) aus Beispiel 6 und 1,00 g (7,2 mmol, 1,0 m%) Salicylsäure eingewogen und gerührt. Die Lösung wird mit einer Geschwindigkeit von 1,5 mL/min auf den Dünnschichtverdampfer aufgetragen. Während der Reaktionsdurchführung werden am Kopf der Kolonne Leichtsieder abdestilliert. Das Produkt 3,7-Dimethylocta-2,6-dienal wird im Sumpf innerhalb des Auffangkolbens angereichert. Nach Beendigung der Reaktion wird das Gerät abgekühlt und entspannt. Die Reaktionslösung im Auffangkolben wird mit 100 mL *tert-*Butyl-methylether versetzt und mit 70 mL einer gesättigten NatriumhydrogencarbonatLösung extrahiert. Nach Phasenseparation wird erneut die organische Phase mit 70 mL einer gesättigten Kochsalz-Lösung gewaschen. Anschließend werden die Phasen getrennt, die organische Phase über Natriumsulfat getrocknet und danach filtriert. Das Lösungsmittel wird anschließend im Vakuum entfernt. Es werden auf diesem Wege 57,1 g Rohprodukt erhalten. Die Rohware wird anschließend über eine KugelrohrApparatur destilliert (Sumpf 75 - 117 °C bei 0,6 mbar). Es werden dabei 49,5 g 3,7-Dimethylocta-2,6-dienal mit einer Reinheit von 74 % isoliert (242 mmol, Ausbeute 73 %).

Die analytischen Daten entsprechen denen der Literatur.

Ein Vergleich der Methoden zeigt, dass mittels dem erfindungsgemäßen Verfahren die gewünschten Produkte in einer deutlich höheren Reinheit hergestellt werden können (siehe Tabelle 1). Dabei ist der Anteil an unerwünschten Nebenprodukten deutlich reduziert, was auf eine selektivere und gleichzeitig schonendere Synthese hindeutet.

**Tabelle 1: Vergleich der Reaktionsprodukte aus Beispiel 2 (erfindungsgemäß) und Beispiel 3 (Reaktivdestillation).**

| **Reaktionsedukt:** Acetal aus Beispiel 1 (Edukt zur Umsetzung): 914,2 g = 2,91 mol | | |
|---|---|---|
| | | |
| | **Beispiel 2 (erfindungsgemäß)** | **Beispiel 3 (Reaktivdestillation)** |
| | | |
| Einsatz | 457,1 g (50 %) | 457,1 g (50 %) |
| Rohprodukt | Ausbeute: 119,4 g; | Ausbeute: 275,06 g; |
| | Reinheit: 72 %-ig | Reinheit: 24,31 %-ig |
| Nach Feindestillation | Ausbeute: 77,4 g; | Ausbeute: 61,5 g; |
| | Reinheit: 98,2 % | Reinheit: 97,8 % |
| | Ausbeute: 221,1 g = 84,3 % d. Th. | Ausbeute: 175,7 g = 66,9 % d. Th. |

## Patentansprüche

1. Verfahren zur Herstellung eines Riechstoffs oder Duftstoffs unter Durchführung von thermischen Umlagerungsreaktionen umfassend die folgenden Schritte:
a) Bereitstellen von mindestens einem Reaktionsedukt und/oder weiteren Reagenzien in einem Dünnschichtverdampfer;
b) Ausführen mindestens einer thermischen Umlagerungsreaktion an den Reaktionsedukten aus Schritt a) im Dünnschichtverdampfer unter destillativen Bedingungen;
c) Erhalt des Riechstoffs oder Duftstoffs.

2. Verfahren nach Anspruch 1, wobei die mindestens eine thermische Umlagerungsreaktion mindestens eine pericyclische Reaktion umfasst.

3. Verfahren nach einem der Ansprüche 1 oder 2, umfassend mindestens eine Umlagerung des Typs der Claisen-Umlagerung, der Cope-Umlagerung und/oder der Prins-Reaktion.

4. Verfahren nach einem der Ansprüche 1 bis 3, umfassend mindestens eine [3,3]-sigmatrope Umlagerung.

5. Verfahren nach einem der Ansprüche 1 bis 4, weiterhin umfassend in Schritt a), die Bereitstellung von mindestens einer Säure als Reagenz.

6. Verfahren nach einem der Ansprüche 1 bis 5, weiterhin umfassend in Schritt a), die Bereitstellung von Salicylsäure als Reagenz für thermische Umlagerungen des Claisen-Typs.

7. Verfahren nach einem der Ansprüche 1 bis 6, wobei das Produkt aus Schritt c) 3-(4-Isopropylcyclohexen-1-yl)propanal der Formel **(I)** ist: 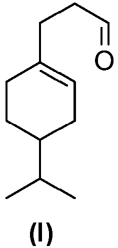 sowie dessen Stereoisomere oder Gemische daraus.

8. Verfahren nach Anspruch 7, wobei die Reaktionsedukte ausgewählt sind aus der Gruppe, umfassend die Acetale der Formeln (**IIa**), (**IIb**) und/oder (**IIc**): 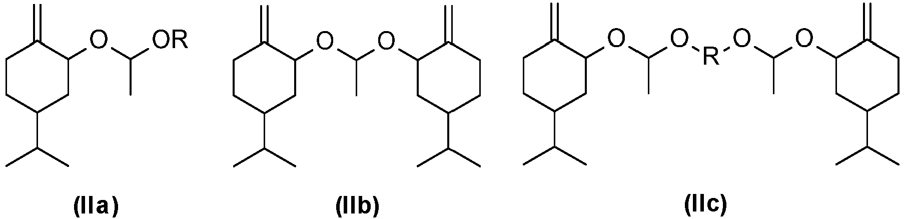 worin R in Verbindungen der Formel (**IIa**) für lineare oder verzweigte Alkylgruppen C₁₋₂₀, Benzylgruppen, Acetylgruppen, Phenylgruppen, -CH₂-CH₂-OCH₃, -CH(CH₃)-CH₂-OCH₃, -(CH₂)₂-O-(CH₂)₂-O-CH=CH₂, -(CH₂)₂-O-(CH₂)₂-O-(CH₂)₂-O-CH=CH₂ oder 2-Methylen-tetrahydrofuran steht, und in Verbindungen der Formel (**IIc**) für lineare oder verzweigte Alkylgruppen C₁₋₂₀, -(CH₂)₂-O-(CH₂)₂-, oder -(CH₂)₂-O-(CH₂)₂-O-(CH₂)₂- steht, sowie deren Stereoisomere, insbesondere Diastereomere und Enantiomere, sowie Mischungen daraus.

9. Verfahren nach Anspruch 7 oder 8, worin die Reste R der Acetale der Formel (**IIa**) Ethylgruppen, Butylgruppen und -(CH₂)₂-O-(CH₂)₂-O-CH=CH₂ sind.

10. Verfahren nach einem der Ansprüche 7 bis 9, weiterhin umfassend einen Verfahrensschritt 0) vor dem Schritt a), wobei in diesem Schritt 0) die Reaktionsedukte der Formeln (**IIa**), (**IIb**) und/oder **(IIc)** über sauer katalysierte Addition von 5-Isopropyl-2-methylen-cyclohexanol an die entsprechenden Vinylether der Formel **(IV)** bereitgestellt werden: 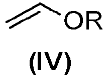 worin R für eine lineare oder verzweigte Alkylgruppe C₁₋₂₀, Benzylgruppen, Acetylgruppen, Phenylgruppen, -CH₂-CH₂-OCH₃, -CH(CH₃)-CH₂-OCH₃, -(CH₂)₂-O-(CH₂)₂-O-CH=CH₂, -(CH₂)₂-O-(CH₂)₂-O-(CH₂)₂-O-CH=CH₂ oder 2-Methylen-tetrahydrofuran steht.

11. Verfahren nach Anspruch 10, wobei als Säurekatalysatoren für die sauer katalysierte Addition in Schritt 0) Lewis-Säuren oder Brønstedt-Säuren eingesetzt werden.

12. Verfahren nach einem der Ansprüche 10 oder 11, worin die Vinylether der Formel **(IV)** in 1,1 bis 5,0-fachem molaren Überschuss eingesetzt werden.

13. Verfahren nach einem der Ansprüche 10 bis 12, worin der im Reaktionsgemisch zurückbleibende Vinylether der Formel **(IV)** destillativ zurückgewonnen wird.

14. Verfahren nach einem der Ansprüche 1 bis 13, wobei das Verfahren als kontinuierlicher Prozess läuft.

15. Verfahren nach einem der Ansprüche 1 bis 14, umfassend die Rückgewinnung von 5-Isopropyl-2-methylen-cyclohexanol, anderen Ausgangsverbindungen und/oder nicht umgelagerten Reaktionsedukten.

## Claims

1. Method for producing an odorant or fragrance by carrying out thermal rearrangement reactions, comprising the following steps:
a) providing at least one reactant and/or further reagents in a thin-film evaporator;
b) carrying out at least one thermal rearrangement reaction on the reactants from step a) in the thin-film evaporator under distillation conditions;
c) obtaining the odorant or fragrance.

2. Method according to claim 1, wherein the at least one thermal rearrangement reaction comprises at least one pericyclic reaction.

3. Method according to any one of claims 1 or 2, comprising at least one rearrangement of the Claisen rearrangement, Cope rearrangement and/or Prins reaction type.

4. Method according to any one of claims 1 to 3, comprising at least one [3,3]-sigmatropic rearrangement.

5. Method according to any one of claims 1 to 4, further comprising, in step a), providing at least one acid as a reagent.

6. Method according to any one of claims 1 to 5, further comprising, in step a), providing salicylic acid as a reagent for thermal rearrangements of the Claisen type.

7. Method according to any one of claims 1 to 6, wherein the product of step c) is 3-(4-isopropylcyclohexen-1-yl)propanal of formula **(I):** 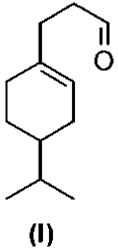 and its stereoisomers, or mixtures thereof.

8. Method according to claim 7, wherein the reactants are selected from the group comprising acetals of formulas (Ila), (IIb) and/or (IIc): 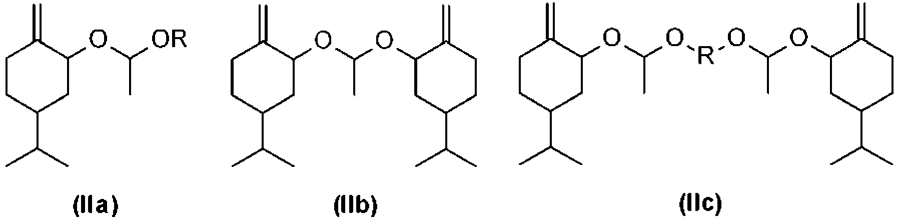 in which R in compounds of formula (IIa) represents linear or branched alkyl groups C₁₋₂₀, benzyl groups, acetyl groups, phenyl groups, -CH₂-CH₂-OCH₃, -CH(CH₃)-CH₂-OCH₃, -(CH₂)₂-O-(CH₂)₂-O-CH=CH₂, -(CH₂)₂-O-(CH₂)₂-O-(CH₂)₂-O-CH=CH₂, or 2-methylenetetrahydrofuran, and in compounds of formula (IIc) represents linear or branched alkyl groups C₁₋₂₀, -(CH₂)₂-O-(CH₂)₂-, or -(CH₂)₂-O-(CH₂)₂-O-(CH₂)₂-, and their stereoisomers, in particular diastereomers and enantiomers, and mixtures thereof.

9. Method according to claim 7 or 8, wherein the radicals R of the acetals of formula (IIa) are ethyl groups, butyl groups, and -(CH₂)₂-O-(CH₂)₂-O-CH=CH₂.

10. Method according to any one of claims 7 to 9, further comprising a method step 0) prior to step a), wherein in this step 0) the reactants of formulas (IIa), (IIb) and/or (IIc) are provided via acid-catalyzed addition of 5-isopropyl-2-methylenecyclohexanol to the corresponding vinyl ethers of formula (IV): 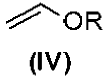 in which R represents a linear or branched alkyl group C₁₋₂₀, benzyl groups, acetyl groups, phenyl groups, -CH₂-CH₂-OCH₃, -CH(CH₃)-CH₂-OCH₃, -(CH₂)₂-O-(CH₂)₂-O-CH=CH₂, -(CH₂)₂-O-(CH₂)₂-O-(CH₂)₂-O-CH=CH₂, or 2-methylenetetrahydrofuran.

11. Method according to claim 10, wherein Lewis acids or Brønstedt acids are used as acid catalysts for the acid-catalyzed addition in step 0).

12. Method according to any one of claims 10 or 11, wherein the vinyl ethers of formula (IV) are used in a 1.1 to 5.0-fold molar excess.

13. Method according to any one of claims 10 to 12, wherein the vinyl ether of formula (IV) remaining in the reaction mixture is recovered by distillation.

14. Method according to any one of claims 1 to 13, wherein the method runs as a continuous process.

15. Method according to any one of claims 1 to 14, comprising recovering 5-isopropyl-2-methylenecyclohexanol, other starting compounds, and/or unrearranged reactants.

## Revendications

1. Procédé de préparation d'une substance odorante ou d'un parfum par exécution de réactions de transposition thermique, comprenant les étapes suivantes :
a) mise à disposition d'au moins un produit de réaction et/ou d'autres réactifs dans un évaporateur à couche mince ;
b) exécution d'au moins une réaction de transposition thermique sur les produits de réaction de l'étape a) dans l'évaporateur à couche mince, dans des conditions de distillation ;
c) obtention de la substance odorante ou du parfum.

2. Procédé selon la revendication 1, où ladite au moins une réaction de transposition thermique comprend au moins une réaction péricyclique.

3. Procédé selon la revendication 1 ou la revendication 2, comprenant au moins une transposition de type transposition de Claisen, transposition de Cope et/ou réaction de Prins.

4. Procédé selon l'une des revendications 1 à 3, comprenant au moins une transposition [3,3]- sigmatropique.

5. Procédé selon l'une des revendications 1 à 4, comprenant en outre, lors de l'étape a), la mise à disposition d'au moins un acide en tant que réactif.

6. Procédé selon l'une des revendications 1 à 5, comprenant en outre, lors de l'étape a), la mise à disposition d'acide salicylique en tant que réactif pour des transpositions thermiques de type Claisen.

7. Procédé selon l'une des revendications 1 à 6, où le produit de l'étape c) est le 3-(4-isopropylcyclohexène-1-yl)propanal de formule (I) : 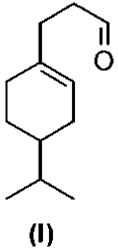 ainsi que ses stéréoisomères ou leurs mélanges.

8. Procédé selon la revendication 7, où les réactifs sont sélectionnés dans le groupe comprenant les acétals de formules (Ila), IIb) et/ou (IIc) : 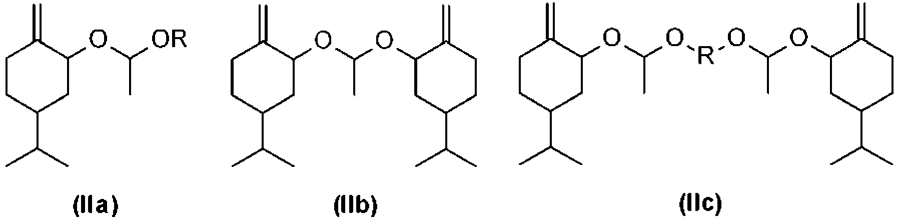 où R, dans des composés de formule (IIa), représente des groupes alkyle linéaires ou ramifiés en C₁₋₂₀, des groupes benzyle, des groupes acétyle, des groupes phényle, -CH₂-CH₂- OCH₃, -CH(CH₃)-CH₂-OCH₃, -(CH₂)₂-O-(CH₂)₂-O-CH=CH₂, -(CH₂)₂-O-(CH₂)₂-O-CH=CH₂ ou le 2-méthylène-tétrahydrofuranne, et, dans des composés de formule (IIc), des groupes alkyle linéaires ou ramifiés en C₁₋₂₀, -(CH₂)₂-O-(CH₂)₂-, ou-(CH₂)₂-O-(CH₂)₂-O-(CH₂)₂-, ainsi que leurs stéréoisomères, en particulier diastéréoisomères et énantiomères, et leurs mélanges.

9. Procédé selon la revendication 7 ou la revendication 8, où les résidus R des acétals de formule (Ila) sont des groupes éthyle, des groupes butyle et -(CH₂)₂-O-(CH₂)₂-O-CH=CH₂.

10. Procédé selon l'une des revendications 7 à 9, comprenant en outre une étape de procédé 0) avant l'étape a), où, lors de ladite étape 0), les produits de réaction de formules (Ha), (IIb) et/ou (IIc) sont mis à disposition au moyen d'une addition à catalyse acide de 5-isopropyl-2-méthylène-cyclohexanol sur les éthers vinyliques correspondants de formule (IV) : 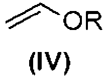 où R représente un groupe alkyle linéaire ou ramifié en C₁₋₂₀, des groupes benzyle, des groupes acétyle, des groupes phényle, -CH₂-CH₂-OCH₃, -CH(CH₃)-CH₂-OCH₃, -(CH₂)₂- O-(CH₂)₂-O-CH=CH₂, -(CH₂)₂-O-(CH₂)₂-O-CH=CH₂ ou le 2-méthylène-tétrahydrofuranne.

11. Procédé selon la revendication 10, où sont utilisés en tant que catalyseurs acides pour l'addition à catalyse acide de l'étape 0) des acides de Lewis ou des acides de Brønstedt.

12. Procédé selon la revendication 10 ou la revendication 11, où les éthers vinyliques de formule (IV) sont utilisés en excès molaire de 1,1 à 5,0 fois.

13. Procédé selon l'une des revendications 10 à 12, où l'éther vinylique de formule (IV) restant dans le mélange réactionnel est récupéré par distillation.

14. Procédé selon l'une des revendications 1 à 13, où le procédé se déroule en tant que processus continu.

15. Procédé selon l'une des revendications 1 à 14, comprenant la récupération du 5-Isopropyl-2-méthylène-cyclohexanol, d'autres composés de départ et/ou de produits de réaction non transposés.
